(19) Europäisches Patentamt
European Patent Office
Office européen des brevets

(11) Publication number: 0 542 363 A2

# (12) EUROPEAN PATENT APPLICATION

(21) Application number: **92203442.6**

(22) Date of filing: **10.11.92**

(51) Int. Cl.[5]: **C07D 211/26**, C07D 211/58, C07D 295/125, C07D 401/04, C07D 401/14, A61K 31/44, A61K 31/445, A61K 31/495

(30) Priority: **14.11.91 GB 9124221**
**16.01.92 GB 9201052**

(43) Date of publication of application:
**19.05.93 Bulletin 93/20**

(84) Designated Contracting States:
**AT BE CH DE DK ES FR GB GR IE IT LI LU MC NL PT SE**

(71) Applicant: **GLAXO GROUP LIMITED**
**Glaxo House, Berkeley Avenue**
**Greenford, Middlesex UB6 0NN(GB)**

(72) Inventor: **Porter, Barry**
**Glaxo Group Research Limited, Park Road**
**Ware, Hertfordshire SG12 0DP(GB)**

Inventor: **Eldred, Colin David**
**Glaxo Group Research Limited, Park Road**
**Ware, Hertfordshire SG12 0DP(GB)**
Inventor: **Kelly, Henry Anderson**
**Glaxo Group Research Limited, Park Road**
**Ware, Hertfordshire SG12 0DP(GB)**
Inventor: **Wheat Croft, James Russell**
**Glaxo Group Research Limited, Park Road**
**Ware, Hertfordshire SG12 0DP(GB)**

(74) Representative: **Brewer, Christopher Laurence et al**
**Glaxo Holdings p.l.c. Glaxo House Berkeley Avenue**
**Greenford, Middlesex UB6 0NN (GB)**

## (54) Piperidineacetic acid derivatives as inhibitors of fibrinogen-dependent blood platelet aggregation.

(57) The invention relates to acetic acid derivatives of formula (I)

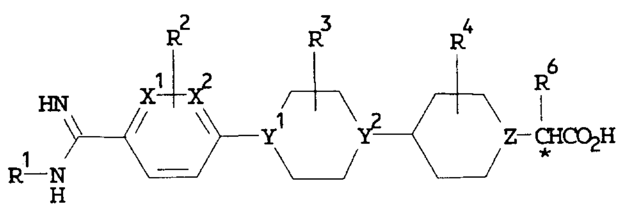

(I)

and pharmaceutically acceptable derivatives thereof and salts and solvates thereof; in which
$X^1$ and $Y^1$, which may be the same or different, represent CH or N;
$X^2$ represents CH or, when $X^1$ represents CH, may also represent N;
$Y^2$ represents N or, when $Y^1$ represents N, may also represent CH;
Z represents N or $N^+R^5$;
$R^1$ represents a hydrogen atom or a hydroxyl, $C_{1-4}$alkyl or 2,2,2- trifluoroethyl group;
$R^2$ represents a hydrogen atom or, when both $X^1$ and $X^2$ represent CH, may also represent a fluorine, chlorine or bromine atom or a $C_{1-4}$alkyl group;
$R^3$ represents a hydrogen atom or, when both $Y^1$ and $Y^2$ represent N, may also represent a $C_{1-4}$alkyl or hydroxymethyl group.
$R^4$ represents a hydrogen atom or , when Z represents N, $R^4$ may also represent a $C_{1-4}$alkyl group;

EP 0 542 363 A2

$R^5$ represents a $C_{1-4}$alkyl or phenyl$C_{1-4}$alkyl group;
$R^6$ represents a hydrogen atom or a $C_{1-4}$alkyl group.

The compounds inhibit fibrinogen − dependent blood platelet aggregation.

This invention relates to acetic acid derivatives, to processes for their preparation, to pharmaceutical compositions containing such compounds and to their use in medicine.

It is widely accepted that the glycoprotein complex Gp IIb/IIIa is the fibrinogen binding site on platelets that mediates the adhesive function required for platelet aggregation and thrombus formation. We have now found a group of non-peptidic compounds which inhibit fibrinogen-dependent platelet aggregation by blocking the binding of fibrinogen to the putative fibrinogen receptor Gp IIb/IIIa complex.

The invention thus provides the compounds of formula (I)

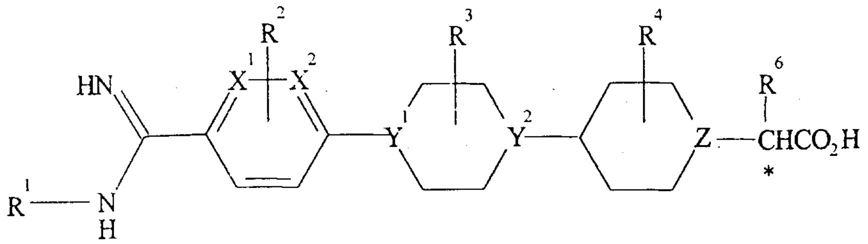

(I)

and salts and solvates thereof, in which

$X^1$ and $Y^1$, which may be the same or different, represent CH or N;

$X^2$ represents CH or, when $X^1$ represents CH, may also represent N;

$Y^2$ represents N or, when $Y^1$ represents N, may also represent CH;

Z represents N or $N^+R^5$,

$R^1$ represents a hydrogen atom or a hydroxyl, $C_{1-4}$alkyl or 2,2,2-trifluoroethyl group;

$R^2$ represents a hydrogen atom or, when both $X^1$ and $X^2$ represent CH, may also represent a fluorine, chlorine or bromine atom or a $C_{1-4}$alkyl group;

$R^3$ represents a hydrogen atom or, when both $Y^1$ and $Y^2$ represent N, may also represent a $C_{1-4}$alkyl or hydroxymethyl group.

$R^4$ represents a hydrogen atom or, when Z represents N, $R^4$ may also represent a $C_{1-4}$alkyl group;

$R^5$ represents a $C_{1-4}$alkyl or phenyl$C_{1-4}$alkyl group;

$R^6$ represents a hydrogen atom or a $C_{1-4}$alkyl group.

In the formulae that follow, the terms "ring -A-", "ring -B-" and "ring -C-" will hereinafter be used to describe certain rings of formula (I):

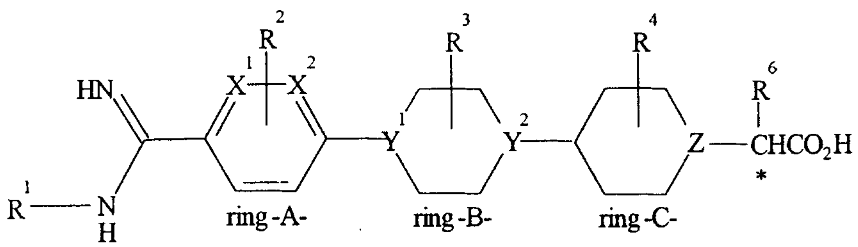

(I)

It will be appreciated that, for pharmaceutical use, the salts referred to above will be the physiologically acceptable salts, but other salts may find use, for example in the preparation of compounds of formula (I) and the physiologically acceptable salts thereof.

It will be further appreciated by those skilled in the art that the compounds of formula (I) in which $R^2$ is other than hydrogen contain at least one chiral centre (shown as * in formula (I)) and that such compounds exist in the form of a pair of optical isomers (i.e. enantiomers). The invention includes all such isomers and mixtures thereof including racemic mixtures.

Suitable physiologically acceptable salts of the compounds of formula (I) include acid addition salts formed with inorganic or organic acids (for example hydrochlorides, hydrobromides, sulphates, phosphates, benzoates, naphthoates, hydroxynaphthoates, p-toluenesulphonates, methanesulphonates, sulphamates, ascorbates, tartrates, salicylates, succinates, lactates, glutarates, glutaconates, acetates, tricarballylates, citrates, fumarates and maleates) and inorganic base salts such as alkali metal salts (for example sodium salts).

Other salts of the compounds of formula (I) include salts formed with trifluoroacetic acid.

It is to be understood that the present invention encompasses all isomers of the compounds of formula (I) and their salts and solvates, including all tautomeric and optical forms, and mixtures thereof (e.g. racemic mixtures).

It is to be further understood that the present invention includes pharmacuetically acceptable derivatives of the compounds of formula (I). By pharmaceutically acceptable derivative is meant any pharmaceutically acceptable ester or salt or solvate of such ester of the compounds of formula (I) or any other compound which upon administration to the recipient is capable of providing (directly or indirectly) a compound of formula (I) or an active metabolite or residue thereof.

It will be appreciated by those skilled in the art that the compounds of formula (I) may be modified to provide pharmaceutically acceptable derivatives thereof at any of the functional groups in the compounds. Of particular interest as such derivatives are compounds modified at the carboxyl or amidine functions.

Thus compounds of interest include carboxylic acid esters of the compounds of formula (I). Examples of such esters include $C_{1-6}$alkyl esters, more preferably $C_{1-3}$alkyl esters, such as ethyl esters.

Other compounds of interest as pharmaceutically acceptable derivatives include benzoylamidine, alkyloxycarbonyl amidine and dialkyloxyphosphinyl amidine derivatives of the compounds of formula (I), which may be prepared by transformation of the amidine group.

It will be appreciated by those skilled in the art that the pharmaceutically acceptable derivatives of the compounds of formula (I) may be derivatised at more than one position.

It will be further appreciated by those skilled in the art that carboxylic acid ester derivatives of formula (I) may be useful as intermediates in the preparation of compounds of formula (I), or as pharmaceutically acceptable derivatives of formula (I), or both.

The term 'alkyl' as a group or part of a group means a straight or branched chain alkyl group, for example a methyl, ethyl, n-propyl, i-propyl, n-butyl, s-butyl or t-butyl group.

In compounds of formula (I), the following meanings are preferred:

$X^1$ and $X^2$ both represent CH;
$Y^1$ and $Y^2$ both represent N;
Z represents N or $N^+R^5$, where $R^5$ represents a methyl or benzyl group;
Z most preferably represents N;
$R^1$ represents a hydrogen atom;
$R^2$ represents a hydrogen, fluorine or chlorine atom, or a $C_{1-4}$alkyl group;
$R^2$ most preferably presents a hydrogen atom;
$R^4$ represents a hydrogen atom;
$R^6$ represents a hydrogen atom or a methyl group; and
$R^6$ most preferably represents a hydrogen atom.

When $R^2$ represents a chlorine or bromine atom or a $C_{1-4}$alkyl group, $R^2$ is preferably in the position meta to the amidine function.

It is to be understood that the present invention covers all combinations of preferred and specific groupings referred to above.

A particularly preferred compound of the invention is 4-[4-[4-(aminoiminomethyl)phenyl]-1-piperazinyl]-1-piperidineacetic acid and physiologically acceptable salts and solvates thereof.

Other preferred compounds of the invention include:

trans-4-[4-[4-(Aminoiminomethyl)phenyl]-1-piperazinyl]-1-[2-(1,1-dimethylethoxy)-2-oxoethyl]-1-methylpiperidinium salts, including physiologically acceptable salts and solvates thereof;

1,1-Dimethylethyl 4-[4-[4-[amino(hydroxyimino)methyl]phenyl]-1-piperazinyl]-1-piperidineacetate and physiologically acceptable salts and solvates thereof;

1,1-Dimethylethyl 4-[4-[4-[imino[(2,2,2-trifluoroethyl)amino] methyl]phenyl]-1-piperazinyl]-1-piperidineacetate and physiologically acceptable salts and solvates thereof;

1,1-Dimethylethyl 4-[4-[6-(aminoiminomethyl)-3-pyridinyl]-1-piperazinyl]-1-piperidineacetate and physiologically acceptable salts and solvates thereof;

1,1-Dimethylethyl 4-[4-[4-(aminoiminomethyl)phenyl]-2-methyl-1-piperazinyl]-1-piperidineacetate and physiologically acceptable salts and solvates thereof;

trans-4-[1-[4-(Aminoiminomethyl)phenyl]-4-piperidinyl]-1-[2-(1,1-dimethylethoxy)-2-oxoethyl]-1-methylpiperidinium salts, including physiologically acceptable salts and solvates thereof;

1,1-Dimethylethyl 4-[4-(aminoiminomethyl)phenyl][1,4'-bipiperidine]-1'-acetate and physiologically acceptable salts and solvates thereof;

1,1-Dimethylethyl 4-[4-[4-(aminoiminomethyl)phenyl]-1-piperazinyl]-1-piperidineacetate and physiologically acceptable salts and solvates thereof;

1,1-Dimethylethyl 4-[4-[4-(aminoiminomethyl)-2-bromophenyl]-1-piperazinyl]-1-piperidineacetate and physiologically acceptable salts and solvates thereof;
1,1 – Dimethylethyl 4 – [4 – [4 – (aminoiminomethyl) – 2 – methylphenyl] – 1 – piperazinyl] – 1 – piperidineacetate and physiologically acceptable salts and solvates thereof;
Ethyl 4 – [4 – [4 – (aminoiminomethyl)phenyl] – 1 – piperazinyl] – α – methyl – 1 – piperidineacetate and physiologically acceptable salts and solvates thereof;
1,1 – Dimethylethyl 1' – [4 – (aminoiminomethyl)phenyl][4,4' – piperidine] – 1 – acetate and physiologically acceptable salts and solvates thereof;
trans – 4 – [4 – [4 – (Aminoiminomethyl)phenyl] – 1 – piperazinyl] – 1 – [2 – (1,1 – dimethylethoxy) – 2 – oxoethyl] – 1 – (phenylmethyl)piperidinium salts; including physiologically acceptable salts and solvates thereof;
cis – 4 – [4 – [4 – (Aminoiminomethyl)phenyl] – 1 – piperazinyl] – 1 – [2 – (1,1 – dimethylethoxy) – 2 – oxoethyl] – 1 – (phenylmethyl)piperidinium salts, including physiologically acceptable salts and solvates thereof;
cis – 1,1 – Dimethylethyl 4 – [4 – [4 – [amino(hydroxyimino)methyl]phenyl] – 1 – piperazinyl] – 3 – methyl – 1 – piperidineacetate and physiologically acceptable salts and solvates thereof;
trans – 4 – [4 – [4 – (Aminoiminomethyl)phenyl] – 1 – piperazinyl] – 1 – (2 – hydroxy – 2 – oxoethyl) – 1 – methylpiperidinium salts, including physiologically acceptable salts and solvates thereof;
4 – [4 – [4 – (Amino(hydroxyimino)methyl]phenyl] – 1 – piperazinyl] – 1 – piperidineacetic acid and physio – logically acceptable salts and solvates thereof;
4 – [4 – [4 – (Imino[(2,2,2 – trifluoroethyl)amino]methyl]phenyl] – 1 – piperazinyl] – 1 – piperidineacetic acid and physiologically acceptable salts and solvates thereof;
4 – [4 – [6 – (Aminoiminomethyl) – 3 – pyridinyl] – 1 – piperazinyl] – 1 – piperidineacetic acid and physiologi – cally acceptable salts and solvates thereof;
4 – [4 – [4 – (Aminoiminomethyl)phenyl] – 2 – methyl – 1 – piperazinyl] – 1 – piperidineacetic acid and phys – iologically acceptable salts and solvates thereof;
trans – 4 – [1 – [4 – (Aminoiminomethyl)phenyl] – 4 – piperidinyl] – 1 – (2 – hydroxy – 2 – oxoethyl) – 1 – methylpiperidinium salts, including physiologically acceptable salts and solvates thereof;
4 – [4 – (Aminoiminomethyl)phenyl][1,4' – bipiperidine] – 1' – acetic acid and physiologically acceptable salts and solvates thereof;
Ethyl 4 – [4 – [4 – (aminoiminomethyl)phenyl] – 1 – piperazinyl] – 1 – piperidineacetate and physiologically acceptable salts and solvates thereof;
4 – [4 – [4 – (Aminoiminomethyl) – 2 – bromophenyl] – 1 – piperazinyl] – 1 – piperidineacetic acid and phys – iologically acceptable salts and solvates thereof;
4 – [4 – [4 – (Aminoiminomethyl) – 2 – methylphenyl] – 1 – piperazinyl] – 1 – piperidineacetic acid and phys – iologically acceptable salts and solvates thereof;
4 – [4 – [4 – (Aminoiminomethyl)phenyl] – 1 – piperazinyl] – α – methyl – 1 – piperidineacetic acid, in the form of a racemic mixture or a single enantiomer, and physiologically acceptable salts and solvates thereof;
1' – [4 – (Aminoiminomethyl)phenyl][4,4' – bipiperidine] – 1 – acetic acid and physiologically acceptable salts and solvates thereof;
trans – 4 – [4 – [4 – (Aminoiminomethyl)phenyl] – 1 – piperazinyl] – 1 – (2 – hydroxy – 2 – oxoethyl) – 1 – (phenylmethyl)piperidinium salts, including physiologically acceptable salts and solvates thereof;
cis – 4 – [4 – [4 – (Aminoiminomethyl)phenyl] – 1 – piperazinyl] – 1 – (2 – hydroxy – 2 – oxoethyl) – 1 – (phenylmethyl)piperidinum salts, including physiologically acceptable salts and solvates thereof;
cis – 4 – [4 – [4 – (Aminoiminomethyl)phenyl] – 1 – piperazinyl] – 3 – methyl – 1 – piperidineacetic acid and physiologically acceptable salts and solvates thereof;
1,1 – Dimethylethyl 4 – [4 – [4 – [[(ethoxycarbonyl)amino]iminomethyl]phenyl] – 1 – piperazinyl] – 1 – piperidineacetate and physiologically acceptable salts and solvates thereof;
4 – [4 – [4 – [[(Ethoxycarbonyl)amino]iminomethyl]phenyl] – 1 – piperazinyl] – piperidineacetic acid and physiologically acceptable salts and solvates thereof;
1,1 – Dimethylethyl 4 – [4 – [4 – [imino[(methoxycarbonyl)amino]methyl]phenyl] – 1 – piperazinyl] – 1 – piperidineacetate and physiologically acceptable salts and solvates thereof;
4 – [4 – [4 – [Imino[(methoxycarbonyl)amino]methyl]phenyl] – 1 – piperazinyl] – 1 – piperidineacetic acid and physiologically acceptable salts and solvates thereof;
1,1 – Dimethylethyl 4 – [4 – [4[[(diethoxyphosphinyl)amino]iminomethyl]phenyl] – 1 – piperazinyl] – 1 – piperidineacetate and physiologically acceptable salts and solvates thereof;
4 – [4 – [4 – [[(Diethylphosphinyl)amino]iminomethyl]phenyl] – 1 – piperazinyl] – 1 – piperidineacetic acid and physiologically acceptable salts and solvates thereof;
1,1 – Dimethylethyl 4 – [4 – [4 – [(benzoylamino)iminomethyl]phenyl] – 1 – piperazinyl] – 1 – piperidineacetate

and physiologically acceptable salts and solvates thereof;
4-[4-[4-[(Benzoylamino)iminomethyl]phenyl]-1-piperazinyl]-1-piperidineacetic acid and physiologically acceptable salts and solvates thereof;
1,1-Dimethylethyl 4-[4-[5-[amino(hydroxyimino)methyl]-2-pyridinyl]-1-piperazinyl]-1-piperidineacetate and physiologically acceptable salts and solvates thereof;
1,1-Dimethylethyl 4-[4-[5-(aminoiminomethyl)-2-pyridinyl]-1-piperazinyl]-1-piperidineacetate and physiologically acceptable salts and solvates thereof; and
4-[4-[5-(Aminoiminomethyl)-2-pyridinyl]-1-piperazinyl]-1-piperidineacetic acid and physiologically acceptable salts and solvates thereof.

Compounds of formula (I) inhibit blood platelet aggregation as demonstrated by studies performed on human gel filtered platelets (GFP) using a Born-type optical aggregometer (Born, G.V., 1962, Nature, 194, 927-929).

In view of their fibrinogen antagonist activity, the compounds of the present invention are of interest for use in human and veterinary medicine, particularly in the treatment or prophylaxis of thrombotic disorders. Particular examples of thrombotic disorders are known in the art and include occlusive vascular diseases such as myocardial infarction, cardiac fatalities, angina, transient ischaemic attacks and thrombotic stroke, arteriosclerosis, vessel wall disease, peripheral vascular disease, nephropathy, retinopathy, postoperative thrombosis, pulmonary embolism, deep vein thrombosis and retinal vein thrombosis. The compounds of the invention are also of interest for use in the prophylaxis of peri- and postoperative complications following organ transplantation (particularly cardiac and renal), coronary artery bypass, peripheral artery bypass, angioplasty, thrombolysis and endarterectomy.

The compounds of the invention may also be useful for the treatment or prophylaxis of other conditions in which the glycoprotein complex Gp IIb/IIIa or other integrin receptors are implicated. Thus, for example, the compounds of the invention may potentiate wound healing and be useful in the treatment of osteoporosis.

The compounds of the invention may also be useful for the treatment of certain cancerous diseases. For example, compounds of the invention may be of use to prevent or delay metastasis in cancer.

According to a further aspect of the invention, we provide a compound of formula (I) or a physiologically acceptable salt or solvate thereof for use in human or veterinary medicine, particularly for use in the treatment or prophylaxis of thrombotic disorders.

According to another aspect of the invention, we provide the use of a compound of formula (I) or a physiologically acceptable salt or solvate thereof for the manufacture of a medicament for the treatment or prophylaxis of thrombotic disorders.

According to a further aspect of the invention, we provide a method of treating a human or animal subject suffering from or susceptible to a thrombotic disorder, which method comprises administering to said subject an effective amount of a compound of formula (I) or a physiologically acceptable salt or solvate thereof.

It will be appreciated that the compounds of formula (I) may advantageously be used in conjunction with one or more other therapeutic agents. Examples of suitable agents for adjunctive therapy include thrombolytic agents or any other compound stimulating thrombolysis or fibrinolysis and cytotoxic drugs. It is to be understood that the present invention covers the use of a compound of formula (I) or a physiologically acceptable salt or solvate thereof in combination with one or more other therapeutic agents.

The compounds of formula (I) and their physiologically acceptable salts and solvates are conveniently administered in the form of pharmaceutical compositions. Thus, in another aspect of the invention, we provide a pharmaceutical composition comprising a compound of formula (I) or a physiologically acceptable salt or solvate thereof adapted for use in human or veterinary medicine. Such compositions may conveniently be presented for use in conventional manner in admixture with one or more physiologically acceptable carriers or excipients.

The compounds according to the invention may be formulated for administration in any suitable manner. The compounds may, for example, be formulated for topical administration or administration by inhalation or, more preferably, for oral or parenteral administration.

For oral administration, the pharmaceutical composition may take the form of, for example, tablets, capsules, powders, solutions, syrups or suspensions prepared by conventional means with acceptable excipients.

For parenteral administration, the pharmaceutical composition may be given as an injection or a continuous infusion (e.g. intravenously, intravascularly or subcutaneously). The compositions may take such forms as suspensions, solutions or emulsions in oily or aqueous vehicles and may contain formulatory agents such as suspending, stabilising and/or dispersing agents. For administration by injection these may

take the form of a unit dose presentation or as a multidose presentation preferably with an added preservative.

Alternatively for parenteral administration the active ingredient may be in powder form for reconstitution with a suitable vehicle.

The compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

As stated above, the compounds of the invention may also be used in combination with other therapeutic agents. The invention thus provides, in a further aspect, a combination comprising a compound of formula (I) or a physiologically acceptable salt or solvate thereof together with another therapeutic agent, in particular a thrombolytic agent.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of formula (I) or a physiologically acceptable salt or solvate thereof is used in combination with a second therapeutic agent active against the same disease state the dose of each compound may differ from that when the compound is used alone. Appropriate doses will be readily appreciated by those skilled in the art.

A proposed daily dosage of a compound of formula (I) for the treatment of man is 0.01 mg/kg to 30 mg/kg, which may be conveniently administered in 1 to 4 doses. The precise dose employed will depend on the age and condition of the patient and on the route of administration. Thus, for example, a daily dose of 0.1mg/kg to 10mg/kg may be suitable for systemic administration.

Suitable methods for the preparation of compounds of formula (I) and salts and solvates thereof are described below. In the formulae that follow, $X^1$, $X^2$, $Y^1$, $Y^2$, Z, $R^1$, $R^2$, $R^3$, $R^4$, $R^5$ and $R^6$ are as defined in formula (I) above unless otherwise stated; $R^p$ represents a protecting group; and Hal represents a halogen, e.g. bromine.

Thus, according to a first process (A), compounds of formula (I) in which $R^1$ represents a hydrogen atom may be prepared from compounds of formula (II)

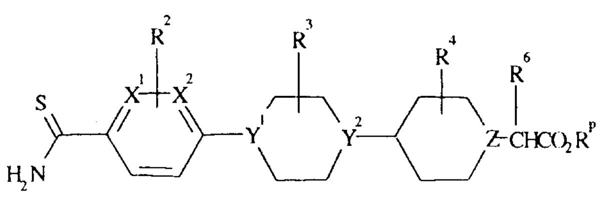

(II)

by treating said compounds of formula (II) with a suitable alkylating agent, followed by reaction with a source of ammonia (e.g. ammonium acetate) in a suitable solvent, such as an alcoholic solvent (e.g. methanol) at an elevated temperature (e.g. reflux), and thereafter removing the carboxylic acid protecting group. The alkylation (e.g. ethylation) may conveniently be effected by employing an appropriate trialkyloxonium salt (e.g. triethyloxonium tetrafluoroborate) in a suitable solvent (e..g. dichloromethane) at room temperature. Alternatively the alkylation (e.g. methylation or benzylation) may conveniently be effected using an alkyl or benzyl halide (e.g. iodomethane) in a suitable solvent such as a ketone (e.g. acetone) at an elevated temperature (e.g. reflux). It will be appreciated that when the alkylation is effected with a $C_{1-4}$alkyl or phenyl$C_{1-4}$alkyl halide alkylation to provide compounds in which Z represents $N^+R^5$ may also result.

According to a further process (B), compounds of formula (I) in which $R^1$ represents a hydroxyl group may be prepared from compounds of formula (III)

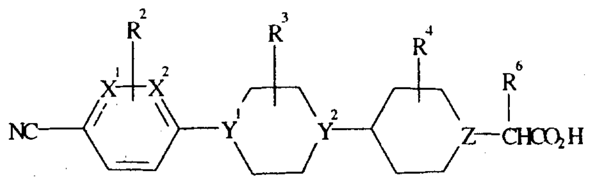 (III)

or protected derivatives thereof by treating said compounds of formula (III) with hydroxylamine or an acid addition salt thereof (e.g. hydroxylamine hydrochloride) in the presence of a suitable base such as an alkali or alkaline earth metal carbonate or bicarbonate (e.g. potassium carbonate) or an alkoxide such as potassium tert-butoxide and in a solvent such as an alcohol (e.g. methanol or tert-butanol), followed, where necessary, by removing any protecting groups present. The reaction with hydroxylamine or an acid addition salt thereof may conveniently be effected at an elevated temperature (e.g. reflux) when a carbonate or bicarbonate is used. When an alkoxide is used the reaction may conveniently be effected at a temperature in the range of about 20° to 80°C.

According to a yet further process (C), compounds of formula (I) in which $R^1$ represents a hydroxyl, $C_{1-4}$alkyl or 2,2,2-trifluoroethyl group may be prepared by treating compounds of formula (II) with a suitable alkylating agent as described in process (A) above followed by reaction of the in situ formed thioimidate with an amine $R^1NH_2$ (where $R^1$ represents hydroxyl, $C_{1-4}$alkyl or 2,2,2-trifluoroethyl), with subsequent removal of the carboxylic acid protecting group. The reaction with the amine $R^1NH_2$ may conveniently be carried out in a suitable solvent such as an alcohol (e.g. methanol) or an ether (e.g. tetrahydrofuran) at an elevated temperature.

According to a still further process (D), compounds of formula (I) may also be prepared by reacting a compound of formula (IV) hereinafter with an alcohol (e.g. ethanol) under acid conditions, followed by treatment of the resulting imidate with a source of ammonia (e.g. ammonium acetate), or an amine $R^1NH_2$ (where $R^1$ is a hydroxyl, $C_{1-4}$alkyl or 2,2,2- trifluoroethyl group), respectively under the conditions described in processes (A) or (C) above, or at ambient temperature, with subsequent removal of the carboxylic acid protecting group.

According to another process (E), compounds of formula (I) may be prepared by interconversion, utilising other compounds of formula (I) as precursors. Thus, for example, compounds of formula (I) in which $R^1$ represents a hydrogen atom may be prepared from corresponding compounds of formula (I) in which $R^1$ represents a hydroxyl group by catalytic hydrogenation in a solvent such as an alcohol (e.g. ethanol), or acetic acid preferably in the presence of acetic anhydride. Suitable catalysts include Raney Nickel or conventional palladium, platinum or rhodium catalysts.

According to yet another process (F), compounds of formula (I) in which $R^1$ is hydrogen, $C_{1-4}$alkyl or 2,2,2-trifluoroethyl and ring -C- represents

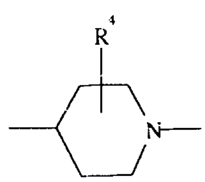

may conveniently be prepared by hydrogenating compounds of formula (XII)

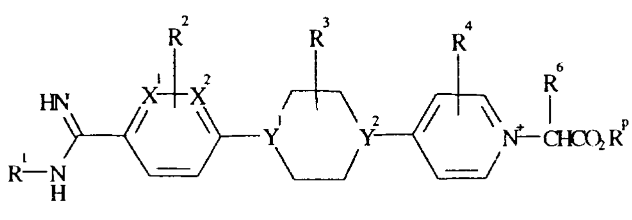 (XII)

(where $R^1$ is hydrogen, $C_{1-4}$alkyl or 2,2,2-trifluoroethyl) at an elevated pressure and in the presence of a platinum catalyst (e.g. $PtO_2$), and thereafter removing the carboxyl protecting group. The reaction may conveniently be effected in a solvent such as an alcohol (e.g. ethanol), and optionally in the presence of an acid, such as hydrochloric acid.

Another process (G) for preparing compounds of formula (I) comprises deprotecting protected derivatives of compounds of formula (I). In a particular embodiment of this process compounds of formula (I) may be prepared from protected carboxyl derivatives of compounds of formula (I).

Suitable carboxyl protection groups include, for example, those described in 'Protective Groups in Organic Synthesis' by Theodora W. Green, second edition, (John Wiley and Sons, 1991) which also describes methods for the removal of such groups. Particular carboxyl protecting groups include, for example, carboxylic acid ester groups such as carboxylic acid alkyl or aralkyl esters, for example where the alkyl or aralkyl portion of the ester function is methyl, ethyl, tert-butyl, methoxymethyl, benzyl, diphenylmethyl, triphenylmethyl or p-nitrobenzyl. When the ester is an unbranched alkyl (e.g. methyl) ester deprotection may be effected under conditions of acid hydrolysis, for example using hydrochloric acid. Tert-butyl and triphenyimethyl ester groups may be removed under conditions of moderate acid hydrolysis, for example using formic or trifluoroacetic acid at room temperature or using hydrochloric acid in acetic acid. Benzyl, diphenylmethyl and nitrobenzyl ester groups may be removed by hydrogenolysis in the presence of a metal catalyst (e.g. palladium).

When a particular isomeric form of a compound of formula (I) is desired, for example where the compound is a quaternary ammonium salt, the required isomer may conveniently be separated using preparative high performance liquid chromatography (h.p.l.c.) applied to the final products of processes (A)-(G) above or applied prior to any final deprotection step in said processes.

Compounds of formula (II) may be prepared from compounds of formula (IV)

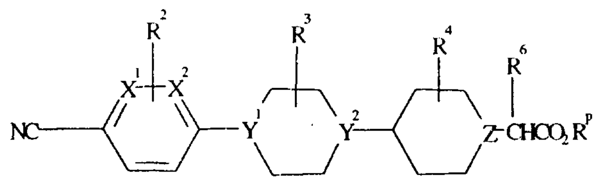

(IV)

by treating said compounds of formula (IV) with hydrogen sulphide. The reaction may conveniently be carried out in a solvent such as dimethylformamide or pyridine and in the presence of an organic base such as an amine (e.g. triethylamine).

Unprotected compounds of formula (III) may also be prepared from compounds of formula (IV) by removing the carboxylic acid protecting group $R^p$ according to the method described in process (E) hereinabove.

Compounds of formula (IV) in which ring -C- represents

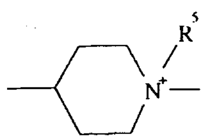

may conveniently be prepared from corresponding compounds of formula (IV) in which ring -C- represents

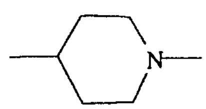

by alkylation according to the method described in process (A) above.

Compounds of formula (IV) in which ring -B- represents

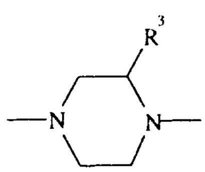

or

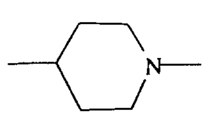

and ring $-C-$ represents

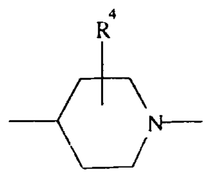

may be prepared by reacting compounds of formula (V)

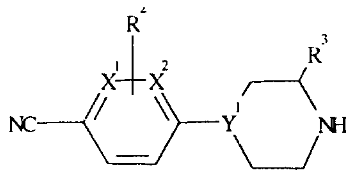 (V)

with compounds of formula (VI)

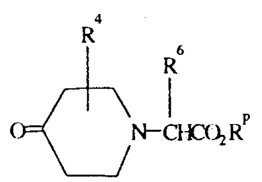 (VI)

under reducing conditions.

Thus, for example, a mixture of compounds of formulae (V) and (VI) may be treated with a reducing agent such as a metal borohydride in the presence of a suitable acid and in a suitable solvent at about room temperature. The reduction may conveniently be carried out using sodium cyanoborohydride in a solvent such as an alcohol (e.g. methanol) in the presence of an acid (e.g. hydrochloric acid) and preferably also with molecular sieves. Alternatively, the reduction may be effected using sodium triacetoxyborohydride in a solvent such as tetrahydrofuran or dichloromethane in the presence of an acid (e.g. acetic acid).

Compounds of formula (VI) may be prepared from an N-protected (e.g. N-benzyl protected) piperidin-4-one, optionally substituted by $C_{1-4}$alkyl, by removal of the protecting group followed by treatment with a reagent $HalCHR^6CO_2R^p$ preferably in the presence of a suitable base such as an alkali metal carbonate or bicarbonate (e.g. potassium carbonate) and in a solvent such as a nitrile (e.g. acetonitrile), conveniently at an elevated temperature (e.g. reflux). When the protecting group is an aralkyl group such as benzyl the removal of the protecting group may be effected by hydrogenolysis in the presence of a suitable transition metal catalyst such as a palladium catalyst (e.g. $Pd(OH)_2$):

Compounds of formula (IV) in which ring $-B-$ represents

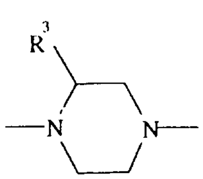 and ring -C- represents 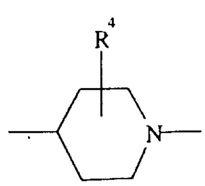

may be prepared by reacting compounds of formula (VII)

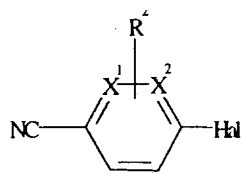 (VII)

with compounds of formula (VIII)

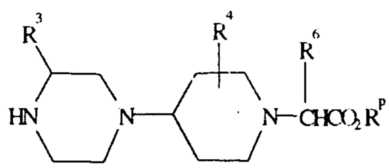 (VIII)

in the presence of a base such as an alkali or alkaline earth metal carbonate or bicarbonate (e.g. sodium bicarbonate) and in a suitable solvent such as an aprotic polar solvent (e.g. dimethylformamide, acetonitrile or dimethylsulphoxide), conveniently at an elevated temperature.

Compounds of formula (VIII) may be prepared from compounds of formula (VI) by reacting said compounds of formula (VI) with a suitable piperazine derivative, optionally protected (e.g. N-benzyl protected), under reducing conditions, for example as described above for the reaction between compounds of formulae (V) and (VI), followed, where appropriate, by the removal of any N-protecting group present using conventional conditions, for example as described above.

Compounds of formula (IV) in which ring -B- represents

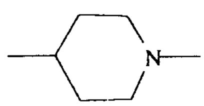 and ring -C- represents 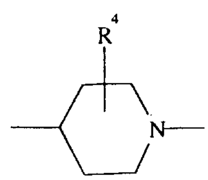

may be prepared from compounds of formula (IX)

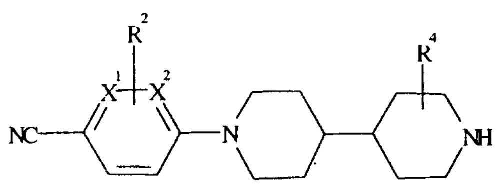 (IX)

by treating said compounds of formula (IX) with a reagent $HalCHR^6CO_2R^p$ under the conditions described above for preparing compounds of formula (VI).

Compounds of formula (IX) may be prepared by reacting a compound of formula (VII) with a compound of formula (X)

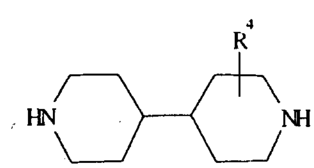 (X)

under the conditions described above for preparing compounds of formula (IV) from compounds of formula (VII). The compound of formula (X) in which $R^4$ is hydrogen is a known compound. Compounds of formula (X) where $R^4$ is alkyl may be prepared by hydrogenation of compounds of formula (XI)

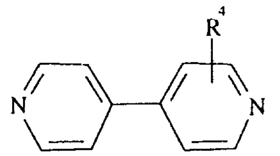 (XI)

Compounds of formula (XI) may be prepared by reacting a 4-halopyridine derivative with 4-pyridylboronic acid, preferably in the presence of a suitable transition metal catalyst such as a palladium catalyst [e.g. tetrakis(triphenylphosphine)palladium(0)] and a suitable base such as an alkali metal carbonate (e.g. sodium carbonate). The reaction may conveniently be effected in a solvent such as an aqueous ether (e.g. aqueous 1,2-ethanediol dimethyl ether).

Compounds of formula (XII) above in which $R^1$ is hydrogen may be prepared from compounds of formula (XIII)

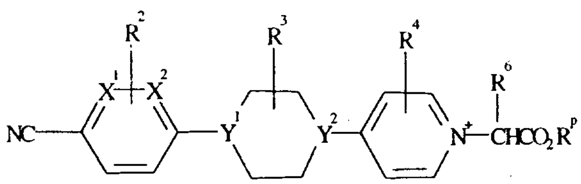 (XIII)

under the conditions described above for preparing compounds of formula (I) from compounds of formula (IV) via compounds of formula (II).

Compounds of formula (XII) in which $R^1$ is $C_{1-4}$alkyl or 2,2,2-trifluoroethyl may also be prepared from compounds of formula (XIII) under the conditions described in processes (C) or (D) above.

Compounds of formula (XIII) may be prepared from compounds of formula (XIV)

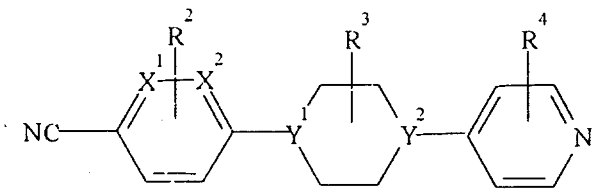 (XIV)

by treatment with a reagent $HalCHR^6CO_2R^p$ under the conditions described above for preparing compounds of formula (VI), except that the reaction is conveniently effected in the absence of a base.

Compounds of formula (XIV) in which ring -B- represents

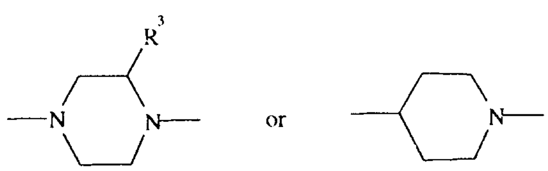

may be prepared by reacting compounds of formula (V) with a suitable 4-halopyridine in a solvent such as an alcohol (e.g. butanol).

Compounds of formula (XIV) in which ring -B- represents

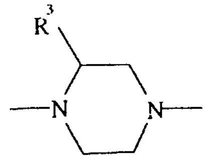

may be prepared by reacting compounds of formula (VII) with compounds of formula (XV)

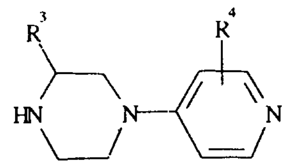

(XV)

under the conditions described above for the reaction between compounds of formulae (VII) and (VIII).

Compounds of formula (XV) may be prepared by reacting a suitable 4-halopyridine with a suitable piperazine under the conditions described above for preparing compounds of formula (IV) from compounds of formula (VII).

Compounds of formula (V) in which ring -B- represents

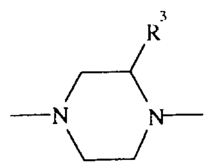

may be prepared by reacting a piperazine

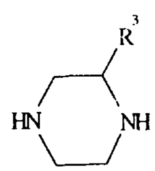

with a compound of formula (VII) under basic conditions. Suitable bases include alkali or alkaline earth metal carbonates or bicarbonates such as sodium bicarbonate or potassium carbonate. The reaction may conveniently be effected in a solvent such as dimethylformamide or dimethylsulphoxide at an elevated temperature (e.g. 100° -200° C).

Compounds of formula (V) in which ring -B- represents

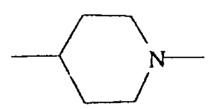

may be prepared from compounds of formula (XVI)

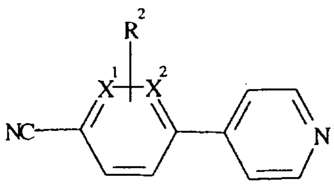

(XVI)

under suitable reducing conditions. Conveniently, compounds of formula (XVI) are treated with benzyl bromide in an alcoholic solvent (e.g. ethanol) or a halogenated hydrocarbon (e.g. dichloromethane) at an elevated temperature to provide a salt of formula (XVII)

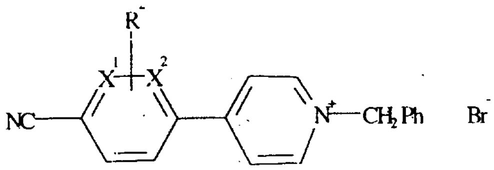 (XVII)

which is reduced, for example using a borohydride reducing agent such as sodium borohydride in a suitable solvent such as an alcohol (e.g. ethanol) or dimethylformamide or a mixture of such solvents to a compound of formula (XVIII)

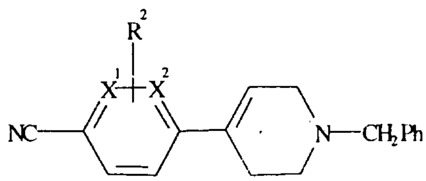 (XVIII)

Removal of the benzyl group and reduction of the double bond from a compound of formula (XVIII) provides the desired compounds of formula (V). The removal of the benzyl group may conveniently be effected by hydrogenolysis in the presence of a palladium catalyst such as $Pd(OH)_2$ – on – carbon, or by reaction with 1 – chloroethyl chloroformate in the presence of a base such as 'proton sponge' followed by treatment with methanol. The reduction of the double bond may conveniently be effected by hydrogenation in the presence of a platinum catalyst such as platinum – on – carbon or platinum oxide or a palladium catalyst such as palladium hydroxide – on – carbon and optionally in the presence of an acid (e.g. hydrochloric acid).

Compounds of formula (IV) in which ring – C – represents

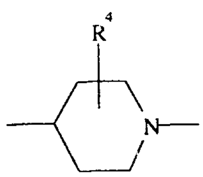

may be prepared from compounds of formula (XIII) by reduction and hydrogenation under the conditions described just above for the reduction and hydrogenation of compounds of formulae (XVII) and (XVIII).

Compounds of formula (XVI) may be prepared by converting compounds of formula (VII) to the corresponding boronic acids of formula (XIX)

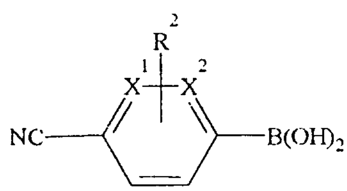 (XIX)

under conventional conditions, and thereafter reacting said compounds of formula (XIX) with a 4 – halopyridine such as 4 – bromopyridine under the conditions described above for preparing compounds of formula (XI).

Compounds of formula (XVI) may also be prepared by reacting an appropriate compound of formula (VII) in which Hal is a bromine atom with a compound of formula (XX)

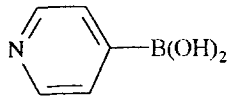 (XX)

under the boronic acid coupling conditions described previously.

The compound of formula (XX) is a known compound described by W. J. Thompson et al. in J. Org. Chem., 1988, 53, 2052.

Compounds of formula (VII) are either known compounds or may be prepared from the known compounds of formula (VII) using conventional chemistry.

Compounds of formula (I) or intermediates thereto in which ring −A− represents a $C_{1-4}$alkyl substituted phenyl group may conveniently be prepared by modification of the corresponding compound in which ring −A− represents a bromo substituted phenyl group. Thus, for example, the bromo substituted intermediate may be treated with a zinc reagent RZnBr (where R is $C_{1-4}$alkyl) in the presence of a palladium catalyst such as 1chloro[1,1'−bis(diphenylphosphino)ferrocene]palladium (II). Alkylation may also be effected using a tin reagent $R_4Sn$ (where R is $C_{1-4}$alkyl) in the presence of a palladium catalyst such as bis (triphenylphosphine)benzylpalladium chloride.

Compounds of formula (IV) in which ring −B− represents

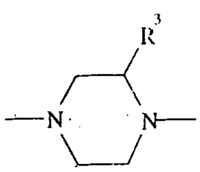 or 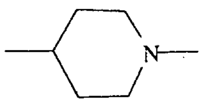 and ring -C- represents 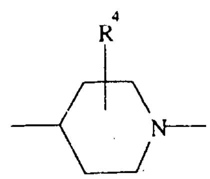

may also be prepared by treating a compound of formula (XXI)

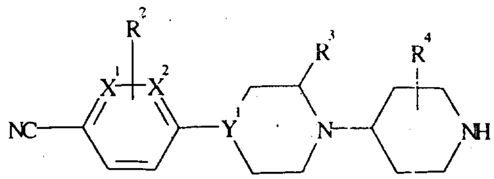 (XXI)

with a reagent $HalCHR^6CO_2R^p$ under the conditions described above for preparing compounds of formula (VI).

Compounds of formula (XXI) may be prepared by reacting compounds of formula (V) with an N−protected piperidin−4−one, optionally substituted by $C_{1-4}$alkyl, under reducing conditions (for example as described above for the reaction between compounds of formulae (V) and (VI)), followed by removing the N−protecting group. Suitable protecting groups include $-CO_2Alk$ (where Alk is an alkyl group such as t−butyl), or aralkyl, for example benzyl. The former protecting group may be removed by acid hydrolysis (e.g. using trifluoroacetic acid at about room temperature), the latter under the conditions described above for the removal of the benzyl group from compounds of formula (XVIII).

Compounds of formula (IV) in which ring −A− represents

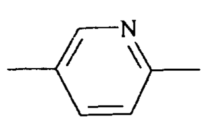

ring −B− represents

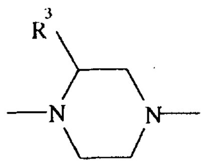 , or 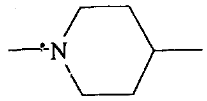

and ring – C – represents

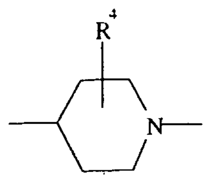

may be prepared by treating a corresponding halo compound of formula (XXII)

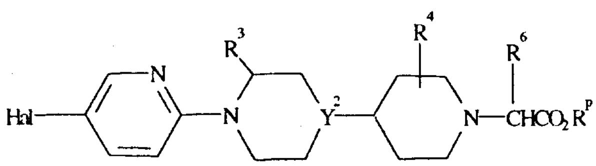 (XXII)

with a suitable inorganic nitrile such as sodium cyanide in the presence of a palladium catalyst [e.g. tetrakis(triphenylphosphine)palladium(0)]. The reaction may conveniently be effected in a solvent such as an aromatic hydrocarbon (e.g. toluene) and preferably in the presence of alumina at an elevated temperature.

Compounds of formula (XXII) may be prepared by reacting a 2,5-dihalopyridine (e.g. 2,5-dibromopyridine) with a compound of formula (VIII) or a compound of formula (XXIII),

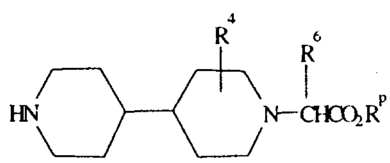 (XXIII)

as appropriate. The reaction may conveniently be effected under the conditions described above for the reaction between compounds of formulae (VII) and (VIII).

Compounds of formula (IX) in which ring – A – represents

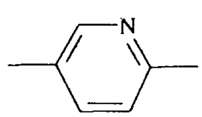

may conveniently be prepared from compounds of formula (XXIV)

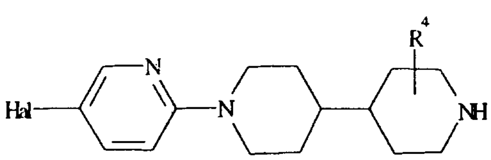 (XXIV)

using the procedure described above for preparing compounds of formula (IV) from compounds of formula (XXII).

Compounds of formula (XXIV) may be prepared by reacting a 2,5 – dihalopyridine (e.g. 2,5 – dibromopyridine) with a compound of formula (X) under the conditions described above for the reaction between compounds of formulae (VII) and (VIII).

Compounds of formula (XIV) in which ring – A – represents

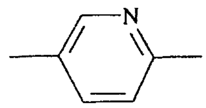

and ring – B – represents

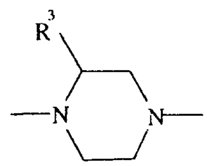

may be prepared from compounds of formula (XXV)

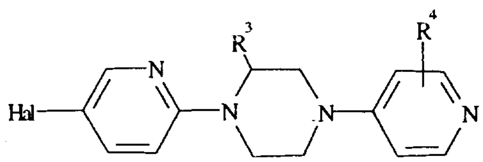
(XXV)

using the procedure described above for preparing compounds of formula (IV) from compounds of formula (XXII).

Compounds of formula (XXV) may be prepared by reacting a 2,5 – dihalopyridine (e.g. 2,5 – dibromopyridine) with a compound of formula (XV) under the conditions described above for the reaction between compounds of formulae (VII) and (VIII).

Compounds of formula (V) in which ring – A – represents

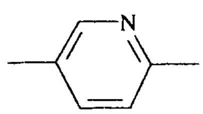

and ring – B – represents

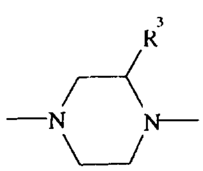

may be prepared from compounds of formula (XXVI)

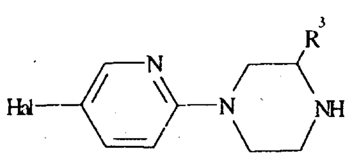
(XXVI)

using the procedure described above for preparing compounds of formula (IV) from compounds of formula (XXII).

Compounds of formula (XXVI) may be prepared by reacting a 2,5-dihalopyridine (e.g. 2,5-dibromopyridine) with a suitable piperazine derivative under the conditions described above for the reaction between compounds of formulae (VII) and (VIII).

Compounds of formula (XXIII) may be prepared by treating a compound of formula (X) with a reagent $HalCHR^6CO_2R^p$ under the conditions described above for preparing compounds of formula (VI).

Compounds of formula (XVI) in which ring -A- represents

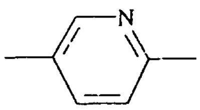

may conveniently be prepared from compounds of formula (XXVII)

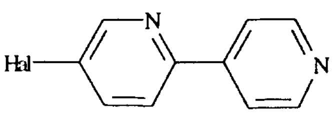

(XXVII)

by reacting said compounds with a suitable inorganic nitrile according to the method described above utilising compounds of formula (XXII).

Compounds of formula (XXVII) may be prepared by reacting a 2,5-dihalopyridine (e.g. 2,5-dibromopyridine) with a compound of formula (XX) under the boronic acid coupling conditions described previously.

Halopyridines and dihalopyridines described above are known in the art. Alkyl substituted halopyridines are either known compounds described in Chem. Pharm. Bull., 1988, 36, 2244 and J. Het. Chem., 1988, 25, 81 or may be prepared according to the methods described therein.

It will be appreciated by those skilled in the art that certain of the procedures described hereinabove for the preparation of compounds of formula (I) or intermediates thereto may not be applicable to some of the possible combinations of rings and substituents.

It will also be appreciated by those skilled in the art that for certain of the methods described hereinabove the desired stereochemistry of the product may be obtained either by commencing with an optically pure starting material or by resolving the racemic mixture at any convenient stage in the synthesis.

Resolution of the final product, an intermediate or a starting material may be effected by any suitable method known in the art: see for example 'Stereochemistry of Carbon Compounds' by E L Eliel (McGraw Hill, 1962) and 'Tables of Resolving Agents' by S H Wilen.

Certain intermediates described above are novel compounds, and it is to be understood that all novel intermediates herein form further aspects of the present invention. Compounds of formula (IV) are key intermediates and represent a particular aspect of the present invention.

Conveniently, compounds of formula (I) are isolated following work-up as acid addition salts, e.g. trifluoroacetate salts. Physiologically acceptable acid addition salts of the compounds of formula (I) may be prepared from the corresponding trifluoroacetate salts by exchange of ion using conventional means, for example by neutralisation of the trifluoroacetate salt using a base such as aqueous sodium hydroxide, followed by addition of a suitable organic or inorganic acid. Inorganic base salts of the compounds of formula (I) may also be prepared from the corresponding trifluoroacetate salts by addition of a suitable strong base such as sodium hydride.

Solvates (e.g. hydrates) of a compound of formula (I) may be formed during the work-up procedure of one of the aforementioned process steps.

The following Preparations and Examples illustrate the invention but do not limit the invention in any way. All temperatures are in °C. Thin layer chromatography (T.l.c.) was carried out on silica plates. System A is dichloromethane-ethanol- 0.880 ammonia. System B is dichloromethane-methanol - 0.880 ammonia. Preparative high performance liquid chromatography (h.p.l.c.) was carried out using a Dynamax 60A C18 8μM 25cm x 41.4mm i.d. column eluted with a mixture of solvents (i) 0.1% trifluoroacetic acid in water and (ii) 0.05% trifluoroacetic acid in acetonitrile. Analytical h.p.l.c. was carried out using a Dynamax 60A C18 8μM 25cm x 4.6mm i.d. column using eluants as for preparative h.p.l.c.

Intermediate 1

4-Piperidinone hydrochloride

N-Benzyl-4-piperidone (10g) was dissolved in absolute ethanol (100ml), treated with dilute hydrochloric acid (2N; 29ml) and hydrogenated at room temperature and pressure over Pearlmann's catalyst (1g) for 18h. The catalyst was removed by filtering through "hyflo" and the solvent was removed in vacuo to leave the title compound (8.59g).
T.l.c. $SiO_2$ (System B 95:5:0.5) Rf 0.33.

Intermediate 2

1,1-Dimethylethyl 4-oxo-1-piperidineacetate

Intermediate 1 (8.5g) was suspended in acetonitrile (100ml) and treated with tert. butyl 2-bromoacetate (11.1ml) and potassium carbonate (17.3g). The mixture was heated under reflux for 24h. The solvent was removed in vacuo and the residue partitioned between water (250ml) and ethyl acetate (3x250ml). The organic layers were washed with brine (100ml), dried ($MgSO_4$), and evaporated in vacuo to leave the title compound (12.1g) as a yellow oil.
T.l.c. $SiO_2$ (System B 95:5:0.5) Rf 0.65.

Intermediate 3

4-(1-Piperazinyl)benzonitrile

4-Chlorobenzonitrile (16.5g) was heated at 180° with piperazine (31g) and sodium carbonate (26.1g) in dimethylsulphoxide (300ml) with stirring under nitrogen for 20h. The mixture was poured into water (1.2l) and extracted with chloroform (3x500ml). The organic layers were washed with water, dried ($MgS0_4$) and evaporated to give the title compound as a yellow oil (23.6g).
T.l.c. $SiO_2$ (System A 83.5:15:1.5) Rf 0.55.

Intermediate 4

1,1-Dimethylethyl 4-[4-(4-cyanophenyl)-1-piperazinyl]-1- piperidineacetate

A solution of Intermediate 3 (3g) in methanol (100ml) was treated with Intermediate 2 (6.84g), 1M hydrogen chloride in ethanol (5ml) and 3 molecular sieves, (~2.5g). Sodium cyanoborohydride (1.06g) was added and the mixture was stirred at room temperature for 48h. The solvent was removed in vacuo and the residual solid was partitioned between water (25ml) and dichloromethane (150ml) and filtered. The layers of the filtrate were separated and the aqueous layer extracted with dichloromethane (3x100ml). The combined organic layers were washed with brine (100ml), dried ($MgSO_4$), filtered and evaporated in vacuo to leave a yellow oil. Purification by "flash" column chromatography using Merck 9385 silica gel and eluting with System B (96:4:0.4) gave the title compound (5.46g) as a white solid.
Analytical h.p.l.c. (gradient profile 10-90% (ii) in 25min) $R_T$ 11.2min.

Intermediate 5

(4-Cyanophenl)boronic acid

A solution of n-butyllithium in hexane (1.58M; 383ml) was added dropwise, at ca. -100° under nitrogen to a stirred solution of 4- bromobenzonitrile (100g) in freshly distilled tetrahydrofuran (1.9L). Addition was complete in 50min and the temperature was maintained at ca. -100° for 15min before triisopropylborate (140ml) was added dropwise between -97° and -96°. Addition was complete in 1h and stirring was continued at ca. -97° for 2.5h. The mixture was allowed to warm to -10° and hydrochloric acid (2M; 352ml) was added dropwise over 10min. The reaction mixture was poured into water (11) and the layers were separated. The organic layer was washed with water (11) and saturated brine (11); dried ($MgSO_4$), filtered and concentrated to give the title compound (60.0g) as a white solid.
N.m.r. ($\delta$, $D_6$-DMSO): 7.82, 7.96 (4H,AA'BB', aromatics), 8.45 (2H,br.s, OH).

Intermediate 6

4-(4-Pyridyl)benzonitrile

A mixture of Intermediate 5 (6g), 4-bromopyridine hydrochloride (9.93g), sodium carbonate (18g) and tetrakis(triphenylphosphine)palladium (0) (2.36g) in a mixture of water (70ml) and 1,2-ethanediol, dimethyl ether (140ml) was heated at reflux, under nitrogen, for 20h. The reaction mixture was allowed to cool to room temperature and was concentrated in vacuo. The residue was partitioned between water (300ml) and ethyl acetate (300ml). The aqueous fraction was extracted with ethyl acetate (300ml) and the combined organic fractions were washed with water (300ml) and saturated brine (300ml), dried ($MgSO_4$), filtered and concentrated in vacuo to give an off-white solid. Purification by dry column 'flash' chromatography using silica gel (Merck 7736) eluting initially with dichloromethane and finally with System A (96:4:0.4) gave the title compound (3.47g) as a colourless solid.

T.l.c. $SiO_2$ (System A 95:5:0.5) Rf 0.5.

Intermediate 7

4-(4-Cyanophenyl)-1-(phenylmethyl)pyridinium bromide

(a) A mixture of Intermediate 6 (7.62g) and benzyl bromide (5.6ml) in absolute ethanol (200ml) was heated at reflux under nitrogen for 4 days. A further quantity of benzyl bromide (1.4ml) was added and the reaction was heated at reflux for a further 20h. The reaction mixture was allowed to cool to room temperature and the solvent was removed in vacuo. The residue was triturated with a mixture of hexane and ethyl acetate (3:1; 200ml) to give the title compound (12.5g) as a light brown solid.

N.m.r. (δ, $D_6$-DMSO) : 5.9 (2H,s, $CH_2$Ph), 7.4-7.7 (5H,m, $CH_2$Ph), 8.16, 8.27, 8.64, 9.38 (8H,2xAA'BB', aromatics).

(b) A mixture of Intermediate 6 (8.41g) and benzyl bromide (7.7ml) in dry dichloromethane (80ml) was heated at reflux under nitrogen for 5h. The mixture was allowed to cool to room temperature and the solvent was removed in vacuo. The solid residue was triturated in a mixture of hexane (100ml) and ethyl acetate (10ml). The solid was filtered off and, washed with hexane (200ml) to give the title compound - (13.6g) as a colourless solid.

N.m.r. (δ, $D_6$-DMSO) : 5.9 (2H,s;$CH_2$Ph), 7.4-7.7 (5H,m;$CH_2$Ph), 8.16, 8.27, 8.64, 9.38 (8H,2xAA'BB'; aromatics).

Intermediate 8

4-[1-(Phenylmethyl)-4-piperidinyl]benzonitrile

A solution of Intermediate 7 (12.4g) in a mixture of absolute ethanol (250ml) and dry dimethylformamide (50ml) was cooled to ca. 0° under nitrogen. Sodium borohydride (2.68g) was added portionwise over 10min. The temperature was maintained at ca. 0° for a further 15min. The reaction mixture was allowed to warm to room temperature and stirring was continued under nitrogen for 18h. The solvent was removed in vacuo, the black residue was pre-adsorbed onto silica gel (Merck 7734) and filtered through a column of silica gel (Merck 9385) eluting with System A (98:2:0.2), and the solvents evaporated to give a brown oil. This oil was hydrogenated in absolute ethanol (250ml) with 20% palladium hydroxide on carbon (1:1 paste with water; 2g) at room temperature and pressure for 36h. The reaction mixture was filtered through hyflo, and the solvent was removed in vacuo to give a brown oil. Purification by dry column 'flash' chromatography using silica gel (Merck 7736) eluting with hexane:dichloromethane:ethanol:0.880 ammonia (100:50:1:0.1 and 50:50:1:0.1) gave the title compound as a yellow oil (4.5g).

T.l.c. $SiO_2$ (System A 98:2:0.2) Rf 0.75.

Intermediate 9

4-(4-Piperidinyl)benzonitrile

(a) A solution of Intermediate 8 (1.13g) and 2M aqueous hydrochloric acid (2ml) in absolute ethanol (150ml) was hydrogenated over 20% palladium hydroxide on carbon (1:1 paste with water; 285mg) at room temperature and pressure for 21h. The mixture was filtered through hyflo, the solvent removed in

vacuo and the solid residue partitioned between ethyl acetate (100ml) and sodium carbonate (2N; 100ml). The aqueous fraction was extracted with ethyl acetate (100ml) and the combined, organic fractions were washed with brine (50ml), dried ($MgSO_4$), filtered and concentrated in vacuo to give the title compound (547mg) as a colourless oil.
T.l.c. $SiO_2$ (System A 95:5:0.5), Rf 0.1.
(b) A solution of Intermediate 8 (4.6g) and 1,8− bis(dimethylamino)naphthalene (0.36g) in dry 1,2−dichloroethane (80ml) was cooled to ca. 0° and treated during 5 min. with 1− chloroethylchloroformate (5.4ml). The reaction mixture was heated at reflux under nitrogen for 4h, cooled to room temperature, and concentrated in vacuo. Methanol (80ml) was added and the mixture was heated at reflux for 4h. The reaction was cooled to room temperature and the solvent was removed in vacuo. The residue was partitioned between ethyl acetate (200ml) and sodium carbonate (2N; 200ml). The aqueous fraction was extracted with ethyl acetate (200ml) and the combined organic fractions were washed with saturated brine (500ml), dried ($MgSO_4$), filtered and concentrated in vacuo to give a red solid. Purification by 'flash' column chromatography using silica gel (Merck 9385) eluting with System A (95:5:0.5 and 90:10:1) gave the title compound (1.49g) as a pale brown solid.
T.l.c. $SiO_2$ (System A 98:2:0.2) Rf 0.2.

Intermediate 10

1−1−Dimethylethyl 4−[4−[4−(aminothioxomethyl)phenyl]−1−piperazinyl]−1−piperidineacetate

Intermediate 4 (1g) was dissolved in dimethylformamide (60ml) and treated with triethylamine (1.09ml). Hydrogen sulphide gas was bubbled through the solution for 20min before the flask was stoppered and stirred at room temperature for 3 days. The mixture was poured into 2N sodium carbonate (250ml) and extracted with dichloromethane (3x300ml). The combined organic layers were washed with brine/water (1:1) (4x300ml) and brine (250ml), dried ($MgSO_4$), and evaporated in vacuo to give the title compound as a yellow solid (630mg).
Analytical h.p.l.c. (gradient profile 10−90% (ii) in 25 min.) $R_T$ 10.3min.

Intermediate 11

1,1−Dimethylethyl 4−[4−(phenylmethyl)−1−piperazinyl]−1− piperidineacetate

1−Benzylpiperazine (8.56g) and Intermediate 2 (10.3g) in methanol (300ml) were treated with 1M hydrogen chloride in ethanol (50ml) and 3 molecular sieves (5g). The mixture was stirred at room temperature and sodium cyanoborohydride (3.1g) added portionwise over 5min. The mixture was stirred at 21° for 40h, filtered through hyflo and evaporated in vacuo. The residue was partitioned between water (150ml) and ethyl acetate (200ml) and the organic phase dried ($Na_2SO_4$) and evaporated in vacuo. The residue was purified by flash chromatography over silica gel (Merck 9385) with System B (95:5:0.2) as eluant to afford the title compound as a colourless crystalline solid (7.80g). T.l.c. $SiO_2$ (System B 89:10:1) Rf 0.4.

Intermediate 12

1,1−Dimethlethyl 4−(1−piperazinyl)−1−piperidineacetate

Intermediate 11 (7.29g) was dissolved in ethanol (200ml) and 1M hydrogen chloride in ethanol (45ml) added followed by water (10ml). The solution was added to pre−hydrogenated 20% palladium hydroxide on carbon (3.1g) in ethanol (50ml) and hydrogenated at room temperature and pressure for 24h (uptake 440ml). The mixture was filtered through hyflo and evaporated in vacuo. The residue in water (50ml) was basified with 2M sodium carbonate to pH>11 and the water removed in vacuo. The residue was triturated with chloroform− methanol (1:1, 200ml) and the filtrate evaporated in vacuo. The residue was taken up in chloroform (100ml), filtered again and evaporated in vacuo to afford the title compound as a colourless solid (5.6g).
T.l.c. $SiO_2$ (System A 29:10:1) Rf 0.23.

Intermediate 13

1,1 – Dimethylethyl 4 – [4 – (6 – cyano – 3 – pyridinyl) – 1 – piperazinyl] – 1 – piperidineacetate

2 – Cyano – 5 – bromopyridine (1.0g.) and Intermediate 12 (1.08g) were dissolved in dimethylsulphoxide (60ml) and water (10ml), sodium bicarbonate (0.5g) was added, and the solution was stirred at 120° for 17h under nitrogen. The mixture was poured into ethyl acetate (250ml), washed with water (3x50ml) and brine (50ml), dried'($Na_2SO_4$) and evaporated in vacuo. The residue was purified by flash chromatography over silica (Merck 9385) with dichloromethane – ethyl acetate (9:1) eluant to remove starting material. Further elution with System B (95:5:0.5) afforded the title compound as a colourless solid (407mg).
T.l.c. $SiO_2$ (System B 90:10:1) Rf 0.4.

Intermediate 14

1,1 – Dimethylethyl 4 – [4 – [6 – (aminothioxomethyl) – 3 – pyridinyl] – 1 – piperazinyl] – 1 – piperidineacetate

Intermediate 13 (401mg) was dissolved in dimethylformamide (50ml) and triethylamine (5ml) and hydrogen sulphide gas was passed through for 0.5h. The dark green solution was stirred at 21° for 2 days then poured into 2M sodium carbonate (100ml) and ethyl acetate (150ml). The mixture was filtered and the aqueous phase further extracted with ethyl acetate (100ml). The combined ethyl acetate extracts were washed with water (3x50ml) and brine (50ml), dried ($Na_2SO_4$) and evaporated in vacuo. The residue was triturated with hexane – ether (3:1, 20ml) to afford the title compound as a yellow solid (145mg).
T.l.c. $SiO_2$ (System B 90:10:1) Rf 0.4.

Intermediate 15

4 – (3 – Methyl – 1 – piperazinyl)benzonitrile

A mixture of 4 – fluorobenzonitrile (6.06g), 2 – methylpiperazine (5.0g) and potassium carbonate (6.93g) in dry dimethylformamide (50ml) was heated at 100° for 2h. The cooled suspension was poured into water (200ml) and diethyl ether (100ml). The aqueous phase was extracted with ether (5x50ml). The combined ethereal extracts were dried ($Na_2SO_4$) and evaporated to an oil. Purification by flash chromatography on silica gel (Merck 9385) using System A (100:8:1) gas eluant gave the title compound as a clear oil (5.75g).
T.l.c. $SiO_2$ (System A 100:8:1) Rf 0.25.

Intermediate 16

1,1 – Dimethylethyl 4 – [4 – (4 – cyanophenyl) – 2 – methyl – 1 – piperazinyl] – 1 – piperidineacetate

Acetyl chloride (0.796g) was added cautiously to dry methanol (50ml). To the solution were added Intermediate 2 (2.34g) and Intermediate 15 (2.0g) in methanol (10ml). Activated 3 molecular sieves (10g) were added and the suspension was stirred at room temperature for 1h. Sodium cyanoborohydride (0.63g) was added cautiously and the mixture was stirred at room temperature for 24h. A second portion of sodium cyanoborohydride (0.063g) was added and the mixture was stirred for a further 18h. The suspension was filtered through "hyflo" and the filtrate was evaporated under reduced pressure. The residue was partitioned between ethyl acetate (100ml) and 8% sodium bicarbonate (100ml). The aqueous phase was extracted with ethyl acetate (50ml). Evaporation of the dried ($MgSO_4$) organic extracts gave an oil which was chromatog – raphed on silica gel (Merck 9385) using System A (400:8:0.5) as eluant. Appropriate eluates were collected and rechromatographed on silica (Merck 9385) using ethyl acetate – methanol (19:1) as eluant to give the title compound as a white solid (0.53g).
T.l.c. $SiO_2$ (ethyl acetate – methanol 19:1) Rf 0.24.

Intermediate 17

1,1 – Dimethylethyl 4 – [4 – [4 – (aminothioxomethyl)phenyl] – 2 – methyl – 1 – piperazinyl] – 1 – piperidineacetate

Intermediate 16 (543mg) was dissolved in dimethylformamide (80ml) – triethylamine (5ml) and hydrogen sulphide gas passed through for 0.5h. The dark green solution was stirred at 21° for 65h and then most of the dimethylformamide was removed in vacuo. The residue was partitioned between 2M sodium carbonate (100ml) and ethyl acetate (200ml). The mixture was filtered and the organic layer of the filtrate dried ($Na_2SO_4$) and evaporated in vacuo. The residue was triturated with hexane – ether (2:1, 25ml) to afford the title compound as a yellow solid (540mg).
T.l.c. $SiO_2$ (System B 89:10:1) Rf 0.33.

Intermediate 18

4 – ([4,4' – Bipiperidin] – 1 – yl)benzonitrile

4,4' – Bipiperidine dihydrochloride (3.2g) was dissolved in dimethylsulphoxide (80ml), potassium carbonate (5.0g) added and the mixture heated at 130° for 20min. 4 – Fluorobenzonitrile (0.93g) was added and the mixture stirred at 130° under nitrogen for 16h. The mixture was cooled, ethyl acetate (250ml) added and the mixture filtered through hyflo. Water (200ml) was added and the organic phase separated. The aqueous phase was re – extracted with ethyl acetate (100ml) and the combined extracts washed with water (2x100ml) and brine (50ml), dried ($Na_2SO_4$) and evaporated in vacuo to afford the title compound as a colourless solid (1.17g).
T.l.c. $SiO_2$ (System A 29:10:1) Rf 0.20.

Intermediate 19

1,1 – Dimethylethyl 1' – (4 – cyanophenyl)[4,4' – bipiperidine] – 1 – acetate

Intermediate 18 (1.17g) in dimethylformamide (50ml) was treated with potassium carbonate (1.2g) and tert – butyl bromoacetate (0.8ml) and the mixture heated at 100° for 16h. Most of the dimethylformamide was removed in vacuo and the residue partitioned between ethyl acetate (150ml) and water (100ml). The organic phase was dried ($Na_2SO_4$) and evaporated in vacuo. The residue in dichloromethane (15ml) was filtered and evaporated in vacuo to give the title compound as a colourless solid (749mg).
T.l.c. $SiO_2$ doped with triethylamine (hexane – ethyl acetate 1:1) Rf 0.24.

Intermediate 20

1,1 – Dimethylethyl 1' – [4 – (aminothioxomethyl)phenyl][4,4' – bipiperidine] – 1 – acetate

Intermediate 19 (745mg) in dimethylformamide (80ml) was treated with triethylamine (5ml) and hydrogen sulphide gas passed through for 30min. The solution was stirred at 21° for 65h and the solvent evaporated in vacuo. The residue was partitioned between ethyl acetate (200ml) and 2M sodium carbonate (100ml) and filtered. The filtered solid was heated under reflux with methanol (50ml) for 0.5h, cooled and filtered. The filtrate and ethyl acetate portion (above) were combined, dried ($Na_2SO_4$) and evaporated in vacuo. Trituration with hexane – ether (1:1, 20ml) afforded the title compound (437mg).
T.l.c. $SiO_2$ doped with triethylamine (dichloromethane – methanol 9:1) Rf 0.5.

Intermediate 21

1,1 – Dimethylethyl 4 – (4 – cyanophenyl)[1,4' – bipiperidine] – 1' – acetate

A mixture of Intermediate 9 (1.49g), Intermediate 2 (1.88g), acetyl chloride (142µl) and activated 3 molecular sieves (7.3g) in dry methanol (80ml) was stirred at room temperature under nitrogen for 18h. Sodium cyanoborohydride (0.51g) was added and the reaction mixture was stirred at room temperature under nitrogen for 22h. The mixture was filtered through hyflo and the solvent was removed in vacuo. The residue was partitioned between ethyl acetate (150ml) and sodium bicarbonate (150ml). The aqueous

fraction was extracted with ethyl acetate (150ml) and the combined organic fractions were washed with brine (200ml), dried ($MgSO_4$), filtered and concentrated in vacuo to give a red oil. Purification by 'flash' column chromatography using silica gel (Sorbsil C60, 20 – 40μM) eluting with System A (98:2:0.2 and 97:3:0.3) gave the title compound (0.424g) as a colourless solid.
T.l.c. $SiO_2$ (System A 98:2:0.2) Rf 0.35.

Intermediate 22

1,1 – Dimethylethyl 4 – [4 – (aminothioxomethyl)phenyl][1,4' – bipiperidine] – 1' – acetate

Hydrogen sulphide gas was bubbled through a solution of Intermediate 21 (0.420g) and triethylamine (5ml) in dry pyridine (25ml) for 30min. The reaction mixture was stirred at room temperature for 18h. The solvent was removed in vacuo and the residue triturated with hexane:diethyl ether (3:1) to give, after filtration, the title compound (0.317g) as a pale yellow solid.
T.l.c. $SiO_2$ (System B 90:10:1) Rf 0.15.

Intermediate 23

3 – Bromo – 4 – (1 – piperazinyl)benzonitrile

3 – Bromo – 4 – fluorobenzonitrile (20.0g) was heated at 120° with piperazine (25.8g) and sodium carbonate (21.2g) in dimethylsulphoxide (250ml) with stirring under nitrogen for 15h. The cooled mixture was poured into water (1 litre) and extracted with dichloromethane (3x500ml). The organic layers were washed with water (3x500ml), dried ($MgSO_4$) and evaporated to give the title compound as a yellow solid (26.6g).
T.l.c. $SiO_2$ (System A 95:5:0.5) Rf 0.15.

Intermediate 24

1,1 – Dimethylethyl 4 – [4 – (2 – bromo – 4 – cyanophenyl) – 1 – piperazinyl] – 1 – pipeidineacetate

A solution of Intermediate 2 (10.0g) in methanol (130ml) was treated with acetyl chloride (942mg) followed by Intermediate 23 (12.5g). 3Å molecular sieves (4g) were added followed by sodium cyanoborohydride (2.95g) and the mixture stirred at room temperature for 18h. The reaction mixture was filtered and the filter cake washed with methanol (50ml). The filtrate was concentrated in vacuo to give a buff solid which was partitioned between ethyl acetate (200ml) and 8% sodium bicarbonate (200ml). The organic extracts were dried ($Na_2SO_4$), concentrated onto silica (Merck 9385), and the title compound obtained by flash chromatography eluting with System B (97:3:0.3) as a yellow solid (4.18g).
Analytical h.p.l.c. (gradient profile 10 – 90% (ii) in 25min) $R_T$ 12.9 min,

Intermediate 25

1,1 – Dimethylethyl 4 – [4 – [4 – (aminothioxomethyl) – 2 – bromophenyl] – 1 – piperazinyl] – 1 – piperidineacetate

Intermediate 24 (1g) was dissolved in pyridine (50ml) and treated with triethylamine (0.9ml). Hydrogen sulphide gas was bubbled through the solution for 25min before the flask was stoppered and stirred at room temperature for 24h.

The solvent was removed in vacuo to leave a yellow oil which as triturated with ether/hexane (1:1) to leave the title compound as a yellow solid (1.05g).
Analytical h.p.l.c. (gradient profile 10 – 90% (ii) in 25min) $R_T$ 12.2 min.

Intermediate 26

1,1 – Dimetliylethyl 4 – [4 – (4 – cyano – 2 – methylphenyl) – 1 – piperazinyl] – 1 – piperidineacetate, trifluoroacetate salt

Intermediate 24 (1g) was suspended in 1,3 – dimethyl – 3,4,5,6 – tetrahydro – 2(1H) – pyrimidinone (40ml) and was treated with tetramethyltin (0.34ml) and tetrakis(triphenylphosphine)palladium (0) (250mg). The mixture was heated at 65° for 18h, poured into water and extracted with dichloromethane (2x50ml). The organic layer was washed with water (4x150ml), dried ($MgSO_4$), filtered and evaporated in vacuo to leave a yellow oil which was purified by preparative h.p.l.c. (gradient profile 10 – 35% (ii) in 12min and 35% (ii) isochratic for 6min) to give after $R_T$ 17.0 min. the title compound as a white solid (395mg).
Analytical h.p.l.c.(gradient profile 10 – 90% (ii) in 25min) $R_T$ 12.3min.

Intermediate 27

1,1 – Dimethylethyl 4 – [4 – [4 – (aminothioxomethyl) – 2 – methylphenyl] – 1 – piperazinyl] – 1 – piperidineacetate trifluoroacetate salt

Intermediate 26 (395mg) was dissolved in pyridine (20ml), treated with triethylamine (0.37ml) and hydrogen sulphide gas was bubbled through the solution for 20min. The flask was stoppered and stirred at room temperature for 24h. The solvent was removed in vacuo to leave the title compound as an orange gum (540mg).
Analytical h.p.l.c. (gradient profile 10 – 90% (ii) in 25min) $R_T$ 11.7min.

Intermediate 28

1,1 – Dimethylethyl 4 – oxo – 1 – piperidinecarboxylate

A mixture of 4 – piperidone monohydrate hydrochloride (25g) and dimethylaminopyridine (2g) in acetonitrile (500ml) and triethylamine (100ml) was cooled in an ice – bath and treated with di – tertiary butyl dicarbonate (35.5g) The mixture was stirred at room temperature under nitrogen overnight. The acetonitrile was removed in vacuo and the residue partitioned between ethyl acetate (400ml) and 1N hydrochloric acid (400ml). The aqueous layer was extracted with ethyl acetate (2x200ml). The combined ethyl acetate extracts were washed with brine (400ml), dried ($MgSO_4$) and evaporated in vacuo to afford the title compound as a white crystalline solid (15.9g).
T.l.c. $SiO_2$ (hexane – ethyl acetate 1:1) Rf 0.4.

Intermediate 29

1,1 – Dimethylethy 4 – [4 – (4 – cyanophenyl) – 1 – piperazinyl] – 1 – piperidinecarboxylate

A solution of Intermediate 28 (15.9g) and Intermediate 3 (14.95g) in dry tetrahydrofuran (500ml) was treated with glacial acetic acid (4.6ml) and sodium triacetoxyborohydride (22g). The reaction was stirred at room temperature under nitrogen for 4.5h. The mixture was concentrated in vacuo and the residue partitioned between 2N sodium carbonate (500ml) and ethyl acetate (500ml). The aqueous layer was extracted with ethyl acetate (2x500ml). The combined organic extracts were washed with brine (750ml) and the solvent removed in vacuo to afford an off – white solid. Purification by flash chromatography on silica gel (Merck 9385) eluting with dichloromethane – methanol (100:1) containing 2% triethylamine gave the title compound as a white solid (19.2g).
T.l.c. $SiO_2$ (System B 90:10:1) Rf 0.67.

Intermediate 30

4 – [4 – (4 – Piperidinyl) – 1 – piperazinyl]benzonitrile trifluoroacetate salt

Intermediate 29 (17.3g) was stirred in trifluoroacetic acid (100ml) and distilled water (10ml) at room temperature under nitrogen for 4.25h. The reaction was concentrated in vacuo and the residue purified by trituration with ether. The resulting white solid was filtered off and dried to afford the title compound –

(24.0g).

| | | | |
|---|---|---|---|
| Analysis Found: | C,44.6; | H,4.62; | N,9.69; |
| $C_{16}H_{22}N_4.2.6C_2HF_3O_2$ requires: | C,44.9; | H,4.37; | N,9.88%. |

Intermediate 31

Ethyl 4–[4–(4–cyanophenyl)–1–piperazinyl]–α–methyl–1–piperidineacetate

A mixture of Intermediate 30 (6g), ethyl–2–bromopropionate (1.5ml) and potassium carbonate (5.88g) in acetonitrile (150ml) was heated under reflux, under nitrogen, for 5h. The acetonitrile was removed in vacuo and the residue partitioned between ethyl acetate (200ml) and water (200ml). The aqueous phase was extracted with ethyl acetate (2x150ml) and the combined organic extracts were washed (water, brine), dried ($Na_2SO_4$) and evaporated in vacuo to give the title compound as a yellow oil which slowly crystallised (4.0g).
T.l.c. $SiO_2$ (System A 95:5:0.5) Rf 0.27.

Intermediate 32

Ethyl 4–[4–[4–(aminothioxomethyl)phenyl]–1–piperazinyl]–α–methyl–1– piperidineacetate

Hydrogen sulphide gas was passed into a solution of Intermediate 31 (4g) and triethylamine (10ml) in pyridine (70ml) for 30min. The vessel was then stoppered and the mixture stirred at room temperature for 3 days. The pyridine was removed in vacuo and the residue triturated with ether (150ml) to give the title compound as a yellow powder (3.05g).
T.l.c. $SiO_2$ (System A 95:5:0.5) Rf 0.05.

Intermediate 33

cis–4–[4–[3–Methyl–1–(phenylmethyl)–4–piperidinyl]–1– piperazinyl]benzonitrile

Intermediate 3 (6.22g) was stirred at room temperature under nitrogen with 1–benzyl–3–methyl–4–piperidone (6.83g) and sodium triacetoxyborohydride (9.2g) in dry dichloromethane (150ml) containing glacial acetic acid (5.67ml) for 19h. The mixture was heated under reflux for 21h, cooled, and treated with 8% aqueous sodium bicarbonate (150ml). The aqueous layer was extracted with dichloromethane (2 x 70ml), and the organic layers were washed with 8% aqueous $NaHCO_3$ (100ml) and water (100ml), dried ($MgSO_4$) and evaporated to give the title compound as a cream solid (11.7g).
T.l.c. $SiO_2$ (System A, 95:5:0.5) Rf 0.7.

Intermediate 34

cis–4–[4–(3–methyl–4–piperidinyl)–1–piperazinyl]benzonitrile

Intermediate 33 (11.3g) was heated under reflux with 1– chloroethyl chloroformate (6.52ml) in 1,2–dichloroethane (300ml) with stirring under nitrogen for 2h. Methanol (80ml) was added, and heating under reflux was continued for 1h. The solvents were evaporated and the residue triturated with dry ether (3 x 80ml) filtered off and dried (8.71g). Trituration with hot ethanol (200ml), followed by cooling, filtration and drying of the residue gave the title compound as a white solid (5.25g).
T.l.c. $SiO_2$ (System A, 78:20:2) Rf 0.2.

Intermediate 35

cis–1,1–Dimethylethyl 4–[4–(4–cyanophenyl)–1–piperazinyl]–3–methyl–1–piperidine acetate

Intermediate 34 (2.0g) was heated under reflux with t–butyl bromoacetate (1.03ml) and anhydrous potassium carbonate (1.47g) in dry acetonitrile (50ml) with stirring under nitrogen for 18h. The solvent was

evaporated and the residue partitioned between ethyl acetate (3 x 50ml) and water (100ml); the organic layers were washed with brine, dried ($MgSO_4$) and evaporated to give the title compound as a light brown solid (1.9g).
T.l.c. $SiO_2$ (System A, 89:10:1) Rf 0.9.

Intermediate 36

1-(5-Bromo-2-pyridinyl)piperazine

A mixutre of 2,5-dibromopyridine (11.7g), piperazine (8.5g) potassium carbonate (6.9g) and dimethylsulphoxide (100ml) was heated at 140° with stirring under nitrogen for 24h. Ethyl acetate (400ml) was added and the mixture washed with water (3 x 150ml), brine (100ml), dried ($Na_2SO_4$) and evaporated in vacuo. The residue was purified by column chromatography using silica gel (Merck 9385) eluting with System B (70:30:1) to give the title compound as a colourless solid (7.64g).
Melting point: 72-74°C.

Intermediate 37

6-(1-Piperazinyl)-3-pyridinecarbonitrile

Intermediate 36 (2g) was heated at 180° with copper (1) cyanide (1.79g) in N-methyl-2-pyrrolidinone (6ml) for 24h. Ammonia solution (20ml) was added and the mixture extracted with ethyl acetate (3x50ml). The combined, organic extracts were washed with water (2x100ml) and brine (2x100ml), dried ($MgSO_4$) and evaporated in vacuo. The residue was purified by flash column chromatography using silica gel (Merck 9385) eluting with System A, 94:6:0.6 to give the title compound as a cream solid (0.330g).
T.l.c. (System A, 90:10:1) Rf 0.45

Intermediate 38

1,1-Dimethylethyl 4-[4-(5-cyano-2-pyridinyl)-1-piperazinyl]-1- piperidineacetate

Intermediate 37 (0.131g), Intermediate 2 (0.35g), acetic acid (0.1ml) and sodium triacetoxyborohydride (0.35g) in tetrahydrofuran (15ml) were stirred at room temperature under nitrogen for 18h. The solvent was evaporated in vacuo and the residue partitioned between ethyl acetate (50ml) and sodium carbonate solution (2N, 10ml). The organic layer was dried ($MgSO_4$), evaporated in vacuo and the residue purified by column chromotography on silica gel (Merck 9385), eluting with System A 96:4:0.4, to give the title compound as a cream solid (0.39g)
T.l.c. (System A, 95:5:0.5) Rf 0.3

Example 1

(a) trans-4-[4-[4-(Aminoiminomethyl)phenyl]-1-piperazinyl]-1-[2-(1,1-dimethylethoxy)-2-oxoethyl]-1-methylpiperidinium trifluoroacetate salt

(b) cis-4-[4-[4-(Aminoiminomethyl)phenyl]-1-piperazinyl]-1-[2-(1,1-dimethylethoxy)-2-oxoethyl]-1-methylpiperidinium trifluoroacetate salt

Intermediate 10 (624mg) was dissolved in acetone (170ml) and treated with methyl iodide (0.927ml). The mixture was heated under reflux for 2½h. The solvent was removed in vacuo to leave a yellow solid which was dissolved in methanol (120ml) and treated with ammonium acetate (345mg). The mixture was heated at 60° for 6h. The solvent was removed in vacuo to leave a yellow solid, which was purified by preparative h.p.l.c. (gradient profile 5-20% (ii) in 10 min. and 20% (ii) isochratic for 8 min.) to give after $R_T$ 16.5 min. the title compound (a) as a white powder (65mg) having a mass spectrum [$MH^+$] of 416, and after $R_T$ 17.0 min the title compound (b) as a pale pink powder (100mg), also having a mass spectrum [$MH^+$] of 416.

Example 2

1,1 – Dimethylethyl 4 – [4 – [4 – [amino(hydroxyimino)methyl]phenyl] – 1 – piperazinyl] – 1 – piperidineacetate

Potassium – t – butoxide (0.123g) was added to a stirred mixture of Intermediate 4 (0.384g) and hydroxylamine hydrochloride (0.076g) in t – butanol (15ml) at 80°. After heating for 18h the residue was partitioned between hot ethyl acetate (50ml) and hot 4% sodium bicarbonate (50ml). The aqueous phase was extracted with hot ethyl acetate (3x50ml). The combined organic extracts were evaporated onto silica gel (Merck 7734). The dried support was added to a column of silica gel (Merck 7734) and eluted with System A (100:8:1). Appropriate eluates were collected and evaporated to yield the title compound (0.11g) as a white solid.
T.l.c. $SiO_2$ (System A 100:8:1) Rf 0.19 and 0.24.

Example 3

1,1 – Dimethylethyl 4 – [4 – [4 – [imino[(2,2,2 – trifluoroethyl)amino] methyl]phenyl] – 1 – piperazinyl] – 1 – piperidineacetate trifluoroacetate salt

Methyl iodide (0.85ml) was added to a stirred suspension of Intermediate 10 (3.0g) in acetone (200ml) and the mixture was heated at reflux for 3h. The solvent was removed in vacuo and the residue dissolved in methanol (200ml). Trifluoroethylamine (1.15ml) was added and the mixture heated at ca. 60° under nitrogen for 5h. The solvent was removed in vacuo to leave a yellow foam which was purified by preparative h.p.l.c. (gradient profile 10 – 70% (ii) in 18 min.) to give after $R_T$ 11.07 min. the title compound as a white powder (182mg). Analytical h.p.l.c. (gradient profile 10 – 90% (ii) in 25 min.) $R_T$ 9.63 min.

Example 4

1,1 – Dimethylethyl 4 – [4 – [6 – (aminoiminomethyl) – 3 – pyridinyl] – 1 – piperazinyl] – 1 – piperidineacetate trifluoroacetate salt

Intermediate 14 (154mg) in acetone (25ml) was treated with iodomethane (30µl) and heated under reflux for 1.5h. Further iodomethane (20µl) was added and after heating under reflux for a further 2h, the solution was homogeneous. The solvent was removed in vacuo, the residue dissolved in methanol (25ml), and ammonium acetate (300mg) added. The solution was heated under reflux for 8h and evaporated in vacuo. The residue was purified by preparative h.p.l.c. (20% (ii) isochratic) to afford the title compound as a colourless solid (46mg).
Analytical h.p.l.c. (gradient profile 10 – 90% (ii) in 25 min.) $R_T$ 8.6min.

Example 5

1,1 – Dimethylethyl 4 – [4 – [4 – (aminoiminomethyl)phenyl] – 2 – methyl – 1 – piperazinyl] – 1 – piperidineacetate trifluoroacetate salt

Intermediate 17 (540mg) in acetone (25ml) was treated with iodomethane (100µl), heated under reflux for 4h, and the solvent evaporated in vacuo. The residue was taken up in methanol (25ml), ammonium acetate (0.5g) added, the solution stirred under reflux for 8h and the solvent evaporated in vacuo. The residue was purified by preparative h.p.l.c. (gradient profile 5 – 20% (ii) in 10 min. and 20% (ii) isochratic for 8 min.) to afford the title compound as a colourless solid.
Analytical h.p.l.c. (gradient profile 10 – 90% (ii) in 25 min.) $R_T$ 9.3min.

Example 6

trans – 4 – [1 – [4 – (Aminoiminomethyl)phenyl] – 4 – piperidinyl] – 1 – [2 – (1,1 – dimethylethoxy) – 2 – oxoethyl] – 1 – methylpiperidinium trifluoroacetate salt

Intermediate 20 (433mg) in acetone (25ml) was treated with iodomethane (70µl) and the mixture was heated under reflux for 3h. The solvent was removed in vacuo, the residue dissolved in methanol (25ml) and ammonium acetate (0.5g) added. The mixture was heated under reflux for 8h, cooled and evaporated in

vacuo. Gradient preparative h.p.l.c. (gradient profile 10 – 40% (ii) over 9min and 40% (ii) isochratic for 9min) afforded the title compound as a colourless solid (61mg).
Analytical h.p.l.c. (gradient profile 10 – 90% (ii) in 25 min.) $R_T$ 11.7 min.

Example 7

1,1 – Dimethylethyl 4 – [4 – (aminoiminomethyl)phenyl][1,4' – bipiperidine] – 1' – acetate trifluoroacetate salt

Intermediate 22 (317mg) and iodomethane (130µl) in acetone (30ml) was heated under reflux under nitrogen for 7h. The mixture was concentrated in vacuo and the residue redissolved in methanol (30ml). Ammonium acetate (176mg) was added and the mixture heated at 60° under nitrogen for 7h. After cooling, evaporation of the solvent in vacuo gave a brown residue, which was purified by preparative h.p.l.c. (gradient profile 5 – 20% (ii) in 10 min. and 20% (ii) isochratic for 8 min.) to give after $R_T$ 15.9 min. the title compound (88mg) as a yellow crystalline solid.
N.m.r. (δ, $D_6$ – DMSO): 1.5 (9H,s,'Bu), 1.9 – 2.4 (8H,m,$CH_2$s), 2.9 – 3.7 (10H,2xm,N – $CH_2$s,N – CH,Ar – CH), 4.05 (2H,brm,N – $CH_2CO_2$'Bu), 7.48 (2H,½AA'BB', aromatic CHs), 7.85 (2H,½AA'BB', aromatic CHs), 9.15, 9.3 (4H,2xbrs, amidine NH), 10.2 (1H,brs,$HO_2CCF_3$).

Example 8

1,1 – Dimethylethyl 4 – [4 – [4 – (aminoiminomethyl)phenyl] – 1 – piperazinyl] – 1 – piperidineacetate

A mixture of Example 2 (0.209g) and 10% palladium on carbon (0.1g) in glacial acetic acid (20ml) was stirred under an atmosphere of hydrogen for 2 days. The suspension was filtered and the filtrate was evaporated under reduced pressure. The residue was partitioned between chloroform (25ml) and 2N sodium carbonate (50ml). The aqueous phase was extracted with chloroform (25ml). The combined organic extracts were dried ($Na_2SO_4$) and evaporated to give a cream solid. Purification by chromatography on silica gel (Merck 7734) using System A (10:8:1) as eluant gave the title compound as a cream powder (0.09g) having an analytical h.p.l.c. (gradient profile 10 – 90% (ii) in 25 min.) $R_T$ 8.65 min.

Example 9

1,1 – Dimethylethyl 4 – [4 – [4 – (aminoiminomethyl) – 2 – bromophenyl] – 1 – piperazinyl] – 1 – piperidineacetate trifluoroacetate salt

Intermediate 25 (1.05g) was dissolved in acetone (70ml) and treated with methyl iodide (0.20ml). The mixture was heated under reflux for 5h and the solvent was removed in vacuo to leave a yellow solid which was dissolved in methanol (50ml) and treated with ammonium acetate (488mg). The mixture was heated at 60° for 6h and the solvent was removed in vacuo to leave a yellow solid. Purification by preparative h.p.l.c. (gradient profile 5 – 20% (ii) in 10min and 20% (ii) isochratic for 8min) gave after $R_T$ 16.3min the title compound as a white solid (105mg).
Mass Spectrometry $[MH]^+$ 481.

Example 10

1,1 – Dimethylethyl 4 – [4 – [4 – (aminoiminomethyl) – 2 – methylphenyl] – 1 – piperazinyl] – 1 – piperidineacetate trifluoroacetate salt

Intermediate 27 (540mg) was heated under reflux with methyl iodide (43µl) in acetone (50ml) for 3h. The solvent was removed in vacuo to leave an orange oil which was dissolved in methanol (50ml), treated with ammonium acetate (163mg) and heated at 60° for 6h. The solvent was removed to leave an orange oil which was purified by preparative h.p.l.c. (gradient profile 5 – 20% (ii) in 10min and 20% (ii) isochratic for 8 min) to give after $R_T$ 16.5 min. the title compound as a cream coloured solid (100mg).
Analytical h.p.l.c. (gradient profile 10 – 90% (ii) in 25min) $R_T$ 9.24min.

Example 11

Ethyl 4-[4-[4-(aminoiminomethyl)phenyl]-1-piperazinyl]-α-methyl-1- piperidineacetate

Methyl iodide (0.35ml) was added to a stirred suspension of Intermediate 32 (2g) in acetone (100ml) and the mixture was heated under reflux under nitrogen for 2.5h. More methyl iodide (0.35ml) was added and the mixture heated under reflux for 1h. The acetone was evaporated and the residue dissolved in methanol (100ml) and treated with ammonium acetate (1.52g). The mixture was heated under reflux for 6h and the methanol removed in vacuo to leave a yellow foam. The foam was taken up in acetonitrile (10ml), water (15ml) and methanol (5ml) and purified by preparative h.p.l.c. (gradient profile 5-20% (ii) in 10min and 20% (ii) isochratic for 8min) to give after $R_T$ 13.4 min. the title compound as a white solid (1.45g).

| | | | |
|---|---|---|---|
| Analysis Found: | C,40.0; | H,4.3; | N,8.0; |
| $C_{21}H_{33}N_5O_2.4.5C_2HF_3O_2$ requires: | C,40.0; | H,4.2; | N,7.8%. |

Example 12

1,1-Dimethylethyl 1'-[4-(aminoiminomethyl)phenyl][4,4'- bipiperidine]-1-acetate

Intermediate 20 (940mg) was suspended in acetone (40ml) and iodomethane (70µl) added. The mixture was stirred at reflux for 1.5h, treated with further iodomethane (70µl) and heated under reflux for a further 1h. A further quantity of iodomethane (50µl) was added and the mixture heated under reflux for a further 1h. The acetone was removed in vacuo and the residue in methanol (60ml) was treated with ammonium acetate (1.5g), heated under reflux for a further 8h, and the solvent evaporated in vacuo. Purification by preparative h.p.l.c. (gradient profile 10-70% (ii) in 18 min) afforded crude product (740mg). Further purification by flash chromatography over silica (Merck 9385, 65g) with System B (19:10:1) eluant afforded the title compound as a colourless solid (108mg).

N.m.r. (δ, $D_6$-DMSO): 8.9 and 8.52 (4H, 2xbrs, amidine NH).
7.7 7.05 (4H, AA'BB', aromatic CH's)
4.03 (2H br d, aromatic $-N-CH_2$ equa.), 3.33 (2Hs, $NCH_2COO$), 3.05 and 2.8 (2H brs + 4H br t, rest $-CH_2N$), 2.1 (2H br m, 2x-CH) and 1.9 - 0.9 (8Hm, rest).

Example 13

(a) trans-4-[4-[4-(Aminoiminomethyl)phenyl]-1-piperazinyl]-1-[2-(1,1-dimethylethoxy)-2-oxoethyl]-1-(phenylmethyl)piperidinium trifluoroacetate salt.

(b) cis-4-[4-[4-(Aminoiminomethyl)phenyl]-1-piperazinyl]-1-[2-(1,1-dimethylethoxy)-2-oxoethyl]-1-(phenylmethyl)piperidinium trifluoroacetate salt.

Intermediate 10 (1.84g), was dissolved in acetone (100ml), treated with benzyl bromide (1.57ml) and heated under reflux for 18h. The solvent was removed in vacuo to leave a yellow solid, which was dissolved in methanol (100ml) and treated with ammonium acetate (1.02g). The mixture was heated at 60°C for 6h, and the solvent evaporated to give an orange oil. Purification by preparative by h.p.l.c. (gradient profile 10-30% (ii) in 12 min. and 30% isochratic for 6min) gave after $R_T$ 17.1 min the title compound (a) as a white solid (32mg), and after $R_T$ 17.3 min the title compound (b) as a white solid (92mg).
Analytical h.p.l.c (gradient profile 10-90% (ii) in 25min)
$R_T$ 11.9min (compound (a)), $R_T$ 12.0min (compound (b)).

Example 14

cis-1,1-Dimethylethyl 4-[4-[4-[amino(hydroxyimino)methyl]phenyl]-1-piperazinyl]-3-methyl-1-piperidineacetate

Intermediate 35 (1.5g) was heated under reflux with hydroxylamine hydrochloride (290mg) and potassium t-butoxide (0.45g) in methanol (20ml) with stirring under nitrogen. The hydroxylamine hydrochloride

and potassium t-butoxide were each added in four equal portions after 0, 1.5, 3.5 and 5.5h, and the mixture was heated under reflux overnights The mixture was poured into water (120ml) and the precipitate filtered off, washed with water and dried to give a white solid (1.33g). The solid was triturated with hot toluene (15ml), filtered off while hot, washed with ether, and dried to give the title compound as a white solid (0.902g).

T.l.c. $SiO_2$ (System A, 89:10:1) Rf 0.6.

Example 15

4-[4-[4-(Aminoiminomethyl)phenyl]-1-piperazinyl]-1-piperidineacetic acid trifluoroacetate salt

Example 8 (152mg) was treated with a mixture of trifluoroacetic acid and water (9:1; 50ml). The mixture was stirred at room temperature for 20min before the reagents were removed in vacuo to leave the title compound as a pink solid (120mg).

| | | | |
|---|---|---|---|
| Analysis Found : | C,39.6; | H,4.5; | N,9.2; |
| $C_{18}H_{27}N_5O_2$.3.65$C_2HF_3O_2$ requires : | C,39.9; | H,4.1; | N,9.2%. |

N.m.r. (δ, DMSO) : 9.03, 8.88 (4H,2xbrs,NHs), 7.8. (2H,½AA'BB', aromatic CHs), 7.2 (2H,½AA'BB', aromatic CHs), 3.98 (2H,s,$-CH_2-$ COOH), 2.8-4.0 (13H,m,$CH_2$s, obscured by $H_2O$), 2.22 (2H,brd,N-$CH_2$-$CH_2$ equatorial), 1.95 (2H,brq,N-$CH_2$-$CH_2$ axial).

Example 16

(a) trans-4-[4-[4-(Aminoiminomethyl)phenyl]-1-piperazinyl]-1-(2-hydroxy-2-oxoethyl)-1-methylpiperidiniumtrifluoroacetate salt

(b) cis-4-[4-[4-(Aminoiminomethyl)phenyl]-1-piperazinyl]-1-(2-hydroxy-2-oxoethyl)-1-methylpiperidinium trifluoroacetate salt

Example 1a (58mg) was treated with trifluoroacetic acid and water (9:1; 25ml) and the mixture stirred at room temperature for 20min. The solvent was removed in vacuo to give the title compound as a white solid (42mg).

| | | | |
|---|---|---|---|
| Analysis Found : | C,40.2; | H,4.6; | N,8.6; |
| $C_{19}H_{30}N_5O_2$.3.75$C_2HF_3O_2$ requires : | C,40.4; | H,4.3; | N,8.9%. |

N.m.r. (δ, MeOD) : 7.75 (2H,½AA'BB', aromatic CHs), 7.16 (2H,½AA'BB', aromatic CHs), 4.22 (2H,s,$N^+$-$CH_2$-COOH) 3.88(2H,brd,$N^+$-$CH_2$ equatorial), 3.68 (5H,m,Ar-N-$CH_2$ + N-CH), 3.32-3.42 ( 9H,m+s,$N^+$-$CH_2$ axial, N-$CH_2$ piperazine,N-Me), 2.3-2.42 (4H,m,N$CH_2$- $CH_2$ piperidine).

Example 1b (92mg) was treated with a mixture of trifluoroacetic acid and water (9:1; 25ml) and stirred at room temperature for 25 min. The solvent was removed in vacuo to give the title compound as a pale pink solid (49mg).

| | | | |
|---|---|---|---|
| Analysis Found: | C,41.0; | H,4.5; | N,9.2; |
| $C_{19}H_{30}N_5O_2$.3.5$C_2HF_3O_2$ Requires: | C,41.1; | H,4.5; | N,9.2% |

Example 17

4-[4-[4-[Amino(hydroxyimino)methyl]phenyl]-1-piperazinyl]-1- piperidineacetic acid trifluoroacetate

A solution of Example 2 (0.12g) in trifluoroacetic acid (4.5ml) and water (0.5ml) was stirred at room temperature for 5h. The solution was evaporated under reduced pressure and the residue triturated under isopropyl acetate to yield the title compound as an off-white powder (0.2g).

| | | | |
|---|---|---|---|
| Analysis Found :<br>$C_{18}H_{27}N_5O_3.3C_2HF_3O_2.0.4C_5H_{10}O_2$ requires: | C,42.02;<br>C,41.95; | H,5.01;<br>H,4.60; | N,9.37;<br>N,9.41%. |

Mass spectrum [$MH^+$] 362.

Example 18

4-[4-[4-[Imino[(2,2,2-trifluoroethyl)amino]methyl]phenyl]-1-piperazinyl]-1-piperidineacetic acid trifluoroacetate salt

Example 3 (50mg) in trifluoroacetic acid/water (10:1, 22ml) was stirred at room temperature for 2h. The solvent was removed in vacuo to give the title compound as a foam (37mg).
Analysis Found: C,38.7; H,4.4; N,7.5;
$C_{20}H_{28}N_5O_2F_3.3.75C_2HF_3O_2.0.5C_2H_6O$ (ethanol) requires :
C,39.0; H,4.0; N,8.0%.
Analytical h.p.l.c. (gradient profile 10-90% (ii) in 18 min.) $R_T$5.36 min.

Example 19

4-[4-[6-(Aminoiminomethyl)-3-pyridinyl]-1-piperazinyl]-1-piperidineacetic acid trifluoroacetate salt

Example 4 (44mg) was dissolved in trifluoroacetic acid-water (9:1, 10ml) and the mixture allowed to stand for 4h. The solvents were removed in vacuo and the residue triturated with dry ether to afford the title compound as a colourless solid (42mg).

| | | | |
|---|---|---|---|
| Analysis Found:<br>$C_{17}H_{26}N_6O_2.4C_2HF_3O_2$ requires : | C,37.65;<br>C,37.4; | H,4.0;<br>H,3.8; | N,9.9;<br>N,10.4%. |

Mass spectrum [$MH^+$] 347.2.

Example 20

4-[4-[4-(Aminoiminomethyl)phenyl]-2-methyl-1-piperazinyl]-1-piperidineacetic acid trifluoroacetate salt

Example 5 (252mg) in trifluoroacetic acid-water (9:1, 20ml) was allowed to stand for 2h and the solvents evaporated in vacuo. The residue was triturated with dry ether (2x5ml) to afford the title compound (232mg).
Analysis Found: C,43.0; H,5.3; N,8.6;
$C_{19}H_{29}N_5O_2.3.3C_2HF_3O_2.0.5C_4H_{10}O$ (diethyl ether) requires :
C,42.8; H,4.8; N,9.1%.
Mass spectrum [$MH^+$] 360.2.

Example 21

trans-4-[1-[4-(Aminoiminomethyl)phenyl]-4-piperidinyl]-1-(2-hydroxy-2-oxoethyl)-1-methylpiperidinium trifluoroacetate salt

Example 6 (60mg) was dissolved in trifluoroacetic acid-water (9:1, 10ml) and the solution allowed to stand for 4h. The solvent was removed in vacuo and the residue triturated with dry ether (5ml). Purification by preparative h.p.l.c. (gradient profile 5-20% (ii) over 10min and 20% (ii) isochratic for 8min) afforded after $R_T$ 13.5 min. the title compound as a colourless solid (15mg).

| | | | |
|---|---|---|---|
| Analysis Found: | C,45.0; | H,5.1; | N,8.3; |
| $C_{20}H_{30}N_4O_2.2.9C_2HF_3O_2$ requires | C,45.0; | H,4.8; | N,8.1%. |

N.m.r. (δ, D6-DMSO): 8.9 and 8.6 (4H,2xbrs,amidine NH's), 7.73 (2H,½AA'BB', aromatic CH's), 7.10-(2H,½AA'BB', aromatic CH's), 4.3 (2H,s,N-$CH_2$-COOH), 4.05 (2H,brd,Ar-$NCH_2$ equatorial), 3.7 (2H,brd,$CH_2$-N-$CH_2$ equatorial), approx. 3.4(2H, Ar-N-$CH_2$ axial- obscured), 3.2 (3H,s,N-$CH_3$), 2.82. (2H,brt,$CH_2$-N-$CH_2$ axial), 2-1.1 (10H,m,rest).

Example 22

4-[4-(Aminoiminomethyl)phenyl][1,4'-bipiperidine]-1'-acetic acid trifluoroacetate salt

Example 7 (82mg) was stirred at room temperature under nitrogen in a mixture of trifluoroacetic acid (4.5ml) and water (0.5ml) for 3.5h. The solvents were removed in vacuo to afford the title compound (67mg) as an off-white solid.
N.m.r. (δ, $D_6$-DMSO) : 1.9-2.4 (8H,m,$CH_2$s), 3-4 (10H-$H_2O$ + 2xm,N-$CH_2$s,N-CH,Ar-CH), 4.08 (2H,s,$NCH_2CO2H$), 7.5(2H,½AA'BB', aromatic CHs), 7.83 (2H,½AA'BB', aromatic CHs), 9.25, 9.3 (4H,2xbrs,amidine NHs), 10.3 (1H,brs,$HO_2CCF_3$).
Mass spectrum [$MH^+$] 345.

Example 23

Ethyl 4-[4-[4-(aminoiminomethyl)phenyl]-1-piperazinyl]-1-piperidineacetate trifluoroacetate salt

Example 15 (0.15g) was dissolved in ethanol (70ml) and hydrogen chloride gas was bubbled into the cooled (ice bath) solution for 20min, which was then stirred overnight at room temperature. The solvent was removed in vacuo to yield the title compound as a pale pink solid (100mg).
Mass spectrum [$MH^+$] 374.2.

| | | | |
|---|---|---|---|
| Analysis Found: | C,39.0; | H,4.0; | N,7.7; |
| $C_{20}H_{31}N_5O_2.4.5C_2HF_3O_2$ requires: | C,39.3; | H,4.0; | N,7.9%. |

Example 24

4-[4-[4-(Aminoiminomethyl)-2-bromophenyl]-1-piperazinyl]-1-piperidineacetic acid trifluoroacetate salt

Example 9 (100mg) was treated with a mixture of trifluoroacetic acid and water (9:1, 25ml) and the mixture stirred at room temperature for 2h. The reagents were removed in vacuo to leave a pink oil which was triturated with ether to leave the title compound as a pink solid (80mg).

| | | | |
|---|---|---|---|
| Analysis Found: | C,37.3; | H,4.0; | N,8.7; |
| $C_{18}H_{26}BrN_5O_2.3.2C_2HF_3O_2$ requires: | C,37.1; | H,3.7; | N,8.9%. |

N.m.r. (δ, D6-DMSO) : 9.1,9.3 (4H,2xbrs amidine NHs), 8.13 (1H,d,aromatic CH), 7.88 (1H,dd,aromatic CH), 7.4 (1H,d,aromatic CH), 4.0 (2H,brs,N-$CH_2$-COOH), 3.2-3.8 (13H,m,rest $CH_2$s+CH), 2.28-(2H,brd,$CH_2$-CH equatorial), 1.95 (2H,brddd,$CH_2$-CH axial).

Example 25

4-[4-[4-(Aminoiminomethyl)-2-methylphenyl]-1-piperazinyl]-1-piperidineacetic acid trifluoroacetate salt

Example 10 (97mg) was treated with trifluoroacetic acid and water (9:1, 25ml) and the mixture stirred at room temperature for 3h. The reagents were removed in vacuo to leave an oil which was triturated with ether to leave the title compound as a white solid (58mg).
Analytical h.p.l.c. (gradient profile 10-90% (ii) in 25min) $R_T$3.5min.

| | | | |
|---|---|---|---|
| Analysis Found: | C,41.6; | H,4.6; | N,9.6; |
| $C_{19}H_{29}N_5O_2.3.3C_2HF_3O_2$ requires : | C,41.8; | H,4.4; | N,9.5%. |

Example 26

4-[4-[4-(Aminoiminomethyl)phenyl]-1-piperazinyl]-α-methyl-1- piperidineacetic acid

Example 11 (1.45g) was dissolved in 2N hydrochloric acid (~100ml) and the mixture stirred at ca. 60° for 3 days. The solvent was removed in vacuo leaving the title compound as a pink solid (0.98g).
Mass spectrometry [$MH^+$] 360.
N.m.r. (δ, $D_6$-DMSO) 1.53 (3H,d,Me), 2.28 (2H,m,$CHCH_2$ (ax)), 2.42 (2H,m,$CHCH_2$ (eq)), 3.06-3.72-(11H,m,ring CH+$CH_2$'s), 4.15 (2H,m,$CH_2$N (eq)), 4.23 (1H,q,CH,$CO_2$H), 7.18, 7.83 (4H,AA'BB', arom.), 8.89, 9.13 (2H,2brs,amidine).

Example 27

1'-[4-(Aminoiminomethyl)phenyl][4,4'-bipiperidine]-1-acetic acid, trifluoroacetate salt

A solution of Example 12 (105mg) in trifluoroacetic acid-water (9:1, 15ml) was allowed to stand at 22° for 2.5h. The solvent was removed in vacuo and the residue purified by preparative h.p.l.c. (gradient profile 5-20% (ii) in 10min and 20% (ii) isochratic for 8 min.) to afford the title compound as a colourless solid (27mg).
Mass spectrum [$MH^+$] 345.5

| | | | |
|---|---|---|---|
| Assay Found | C,42.9; | H,4.4; | N,7.9; |
| $C_{19}H_{28}N_4O_2$. 3.25 $CF_3CO_2H$ requires | C,42.8; | H,4.4; | N,7.8%. |

Example 28

trans-4-[4-[4-(Aminoiminomethyl)phenyl]-1-piperazinyl]-1-(2-hydroxy-2-oxoethyl)-1-(phenylmethyl)piperidinium trifluoroacetate salt

Example 13 (a) (30mg) was treated with trifluoroacetic acid and water (1:1) (10ml), and the mixture was stirred at room temperature for 18h. The solvent was removed in vacuo to give the title compound as an off white solid (26mg)
Mass spectrum: [$MH^+$] 436

| | | | |
|---|---|---|---|
| Assay Found | C,45.7; | H,4.5; | N,8.4; |
| $C_{25}H_{24}N_5O_2$.3.5TFA requires | C,46.0; | H,4.5; | N,8.4%. |

Example 29

cis-4-[4-[4-(Aminoiminomethyl)phenyl]-1-piperazinyl]-1-(2-hydroxy-2-oxoethyl)-1-(phenylmethyl)piperidiniumtrifluoroacetate salt

Example 13 (b) (86mg) was treated with trifluoroacetic acid and water (9:1, 10ml), and the mixture was stirred at room temperature for 18h. The solvent was removed in vacuo to give the title compound as a white solid (76mg).
Mass spectrum [$MH^+$] 436

| | | | |
|---|---|---|---|
| Assay Found | C,46.5; | H,4.6; | N,8.5; |
| $C_{25}H_{34}N_5O_2$. 3.25 TFA requires | C,46.9; | H,4.7; | N,8.7.%. |

Example 30

cis-4-[4-[4-Aminoiminomethyl)phenyl]-1-piperazinyl]-3-methyl-1-piperidineacetic acid trihydrochloride salt

Acetic acid (10ml) and acetic anhydride (0.3ml) were added together to a mixture of Example 14 (0.9g) and 5% palladium on carbon (100mg) under nitrogen, and the mixture was hydrogenated at room temperature and pressure until hydrogen uptake had ceased. The mixture was filtered through 5N HCl washed Hi-flow, and the filter cake washed with 5N hydrochloric acid (3 x 10ml). The filtrate was stirred under nitrogen for 18h, giving a dark green solution. The mixture was concentrated to ca. 4ml, and isopropanol (40ml) added, giving a precipitate which was filtered off and washed with isopropanol to give a light green solid (488mg). Recrystallisation from ca. 5:1 isopropanol:water (ca. 30ml) gave the title compound as a light grey powder (148mg).

| | | | |
|---|---|---|---|
| Assay Found: | C,44.1, | H,6.9; | N,13.4; |
| $C_{19}H_{29}N_5O_2$. 3HCl. 2.7$H_2O$ requires: | C,44.1; | H,7.3; | N,13.5%. |

Analytical h.p.l.c. (gradient profile 5-20% (ii) in 10min) $R_T$ 5.6min.

Example 31

1,1-Dimethylethyl 4-[4-[4-[[(ethoxycarbonyl)amino]iminomethyl]phenyl]-1-piperazinyl] -1-piperidineacetate

Sodium hydroxide solution (2N: 3ml) was added to Example 8 (0.7g) and the suspension evaporated to dryness. The solid residue was suspended in dry pyridine (15ml) and ethyl chloroformate (0.214ml) added. After 1h, the solvent was removed in vacuo and the residue purified by preparative h.p.l.c. (gradient profile 10-45% (ii) in 17 min) to give after $R_t$ 12.9 min the title compound as a beige solid (0.03g).
Analytical h.p.l.c. (gradient profile 10-90% (ii) in 25 min) $R_t$ 10.8min.

Example 32

4-[4-[4-[[(Ethoxycarbonyl)amino]iminomethyl]phenyl]-1-piperazinyl]- 1-piperidineacetic acid trifluoroacetate salt

A solution of Example 31 (0.032g), in trifluoroacetic acid (6ml) and water (1ml) was stirred at room temperature under nitrogen for 5h. The solvent was evaporated to give the title compound as a white solid (0.025g).

| | | | |
|---|---|---|---|
| Assay Found: | C,37.3; | H,3.6; | N,6.8. |
| $C_{21}H_{31}N_5O_4$ 4.8 $CF_3$ $CO_2H$ requires | C,37.7; | H,3.7; | N,7.1% |

Mass Spectrum [$MH^+$] 418

Example 33

1,1-Dimethylethyl 4-[4-[4-[imino[(methoxycarbonyl)amino] methyl]phenyl]-1-piperazinyl]-1-piperidineacetate.

A solution of Example 8 (0.4g) in dry dichlomethane (50ml) was treated with methyl chloroformate (0.05ml) and then sodium hydroxide solution (0.1N, 26.4ml) and the mixture was stirred vigorously for 20 min. The organic layer was separated, dried ($MgSO_4$) and evaporated to yield the title compound as a yellow solid (0.3g).
T.l.c. (System A, 90:10:1) Rf 0.7.

Example 34

4-[4-[4-[Imino[(methoxycarbonyl)amino]methyl]phenyl]-1-piperazinyl]-1-piperidineacetic acid trifluoroacetate salt

Example 33 (0.4g) was dissolved in trifluoroacetic acid (18ml) and water (3ml) and the mixture was stirred at room temperature for 1h. The mixture was concentrated in vacuo and the residue purified by preparative h.p.l.c. (gradient profile 5-20% (ii) in 10 min and 20% (ii) isochratic for 8 min) to give after $R_t$12 min the title compound as a beige solid (0.22g).
Analytical h.p.l.c. (gradient profile 10- 90% (ii) in 25 min) $R_t$ 3.55.
Mass Spectrum [$MH^+$] 404

Example 35

1,1-Dimethylethyl 4-[4-[4[[(diethoxyphosphinyl)amino] iminomethyl]phenyl]-1-piperazinyl]-1-piperidineacetate

Sodium hydroxide solution (2N, 20ml) was added to Example 8 (0.9g) and the solution evaporated to dryness. Dimethylformamide (10ml) followed by diethyl cyanophosphate (0.264ml) were added to the solid residue and the solution stirred at room temperature for 15h. The solution was filtered and the filtrate evaporated in vacuo to give the title compound as a red oil (0.6g).
Mass Spectrum [$MH^+$] 538.

Example 36

4-[4-[4-[(Diethylphosphinyl)amino]iminomethyl]phenyl]-1-piperazinyl]-1- piperidineacetic acid trifluoroacetate salt

Example 35 (0.6g) was stirred in trifluoroacetic acid (9ml) and water (1ml) for 2h at room temperature. The mixture was concentrated in vacuo and the residue purified by preparative h.p.l.c. (gradient profile 5-40% (ii) in 17min) to give after $R_T$ 14.4 min the title compound as a foam (0.12g).
Analytical h.p.l.c. (gradient profile 10-90% (ii) in 25mm) $R_T$ 7.9 min.
Mass Spectrum [$MH^+$] 482

Example 37

1,1-Dimethylethyl 4-[4-[4-[(benzoylamino)iminomethyl]phenyl]-1-piperazinyl]-1-piperidineacetate

Example 8 (0.3g) in dichloromethane (20ml) was treated with benzoyl chloride (27µl) and sodium hydroxide solution (0.1N; 14ml), and subsequently stirred vigorously for 2h. The organic layer was separated, dried ($MgSO_4$), and evaporated in vacuo to yield a yellow residue which was purified by

preparative h.p.l.c. (gradient profile 10 – 55% (ii) in 17 min) to give after $R_T$ 13.1 min the title compound as a yellow solid (0.11g)
Mass Spectrum [$MH^+$] 506.

Example 38

4 – [4 – [4 – [(Benzoylamino)iminomethyl]phenyl] – 1 – piperazinyl] – 1 – piperidineacetic acid trifluoroacetate salt

Example 37 (0.1g) was dissolved in trifluoroacetic acid (10ml) and water (1ml) and the mixture was stirred at room temperature for 1h. The solvents were evaporated to give a yellow oil which was purified by preparative h.p.l.c. (gradient profile 10 – 50% (ii) in 11 min) to give after $R_t$ 11.1 min the title compound as a cream solid (0.07g).

| | | | |
|---|---|---|---|
| Assay Found: | C.44.6; | H.4.1; | N,8.0 |
| $C_{25}H_{31}N_5O_3$ $4CF_3CO_2H$ requires | C.44.5; | H.4.0; | N.7.9% |

Analytical h.p.l.c. (gradient profile 10 – 90% (ii) in 25 min) $R_t$ 11.2 min.

Example 39

1,1 – Dimethylethyl 4 – [4 – [5 – [amino(hydroxyimino)methyl] – 2 – pyridinyl] – 1 – piperazinyl] – 1 – piperidineacetate

Intermediate 38 (0.35g) in methanol (20ml) was treated with 4 portions of potassium t – butoxide (0.058g) and hydroxylamine hydrochloride (0.036g) at 2h intervals while the mixture was heated under reflux under nitrogen, and after the final portion had been added, the mixture was heated under reflux for 8h. The solution was cooled, water (10ml) added, and the product filtered off and dried in vacuo to give the title compound as a white solid (0.25g).
T.l.c. (System A. 90:10:1) Rf 0.4;

Example 40

1,1 – Dimethylethyl 4 – [4 – [5 – (aminoiminomethyl) – 2 – pyridinyl] – 1 – piperazinyl] – 1 – piperidineacetate trifluoroacetate salt

A mixture of Example 39 (0.23g), acetic anhydride (78μl) and 10% palladium on carbon (50mg) in glacial acetic acid (6ml) was hydrogenated at room temperature and pressure for 1h. The catalyst was filtered off and the filtrate evaporated in vacuo. Purification by preparative h.p.l.c. (gradient profile 10 – 50% (ii) in 17 min) gave after $R_t$ 10.8 min the title compound (0.12g) as a white solid.
Mass Spectrum [$MH^+$] 402

Example 41

4 – [4 – [5 – (Aminoiminomethyl) – 2 – pyridinyl] – 1 – piperazinyl] – 1 – piperidineacetic acid trifluoroacetate salt

Example 40 (0.1g) was stirred with trifluoroacetic acid (9ml) and water (1ml) at room temperature for 1.5h. The solvent was removed in vacuo to give an oily residue which was purified by preparative h.p.l.c. (gradient profile 5 – 20% (ii) in 10 min and 20% (ii) isochratic for 8 min) to give after $R_t$ 13.0 min the title compound as a white solid (0.02g).
Mass Spectrum [$MH^+$] 347
Analytical h.p.lc. (gradient profile 10 – 90% (ii) in 25 min.) $R_T$ 4.12 min.

Example 42

Biological Data

Inhibition of blood platelet aggregation by compounds of the invention was determined according to the following procedure. Citrated whole blood (1 part 3.8% trisodium citrate : 9 parts blood) was obtained from human volunteers, free of medication for at least 10 days prior to collection. The blood was incubated with 0.1mM aspirin and 0.05µM prostacyclin and then centrifuged at 1000g for 4 minutes (20°C). The supernatant platelet rich plasma (PRP) was further centrifuged at 1300g for 10 minutes (20°C) to sediment the platelets. The supernatant was discarded and the pellet washed with a physiological salt solution (HEPES 5mM, $NaHCO_3$ 12mM, NaCl 140mM, $KH_2PO_4$ 0.74mM, D-Glucose 5.6mM, KCl 2.82mM and BSA 20g/l, pH 7.4) to remove residual plasma. After washing, the pellet was resuspended in physiological salt solution and then applied to a sepharose CL-2B column, pre-equilibrated with physiological salt solution at room temperature. The platelets (GFP) eluted within the void volume and were diluted to approximately 300,000 platelets/µl in buffer. Purified human fibrinogen (Knight L.C. et al, 1981 Thromb. Haemostasis, 46-(3), 593-596) was added to a final concentration of 0.5mg/ml together with $Ca2+$ and $Mg2+$ at 1mM and 0.5mM respectively. Test compounds were incubated in GFP for 5 minutes at 37°C and the platelet aggregating agent adenosine diphosphate (ADP) was added to a final concentration of 1 x $10^{-5}$M. The potency of the compounds may be expressed as an $IC_{50}$ value defined as the concentration of compound required to produce 50% inhibition of platelet aggregation.

The following $IC_{50}$ values were obtained for compounds of the invention:

| Compound Example No. | $IC_{50}$(nM) |
|---|---|
| 15 | 37 |
| 16a | 44 |
| 17 | 5662 |
| 18 | 316 |
| 19 | 76 |
| 20 | 71 |
| 21 | 41 |
| 22 | 71 |
| 23 | 16233 |
| 24 | 342 |
| 25 | 569 |
| 26 | 177 |
| 27 | 32 |
| 28 | 38 |
| 29 | 354 |
| 30 | 23 |
| 32 | 3332 |
| 34 | 1599 |
| 36 | 2683 |
| 38 | 1296 |
| 41 | 30 |

Example 43

Pharmacy Example - Tablets

| | |
|---|---|
| a) Compound of the invention | 5.0mg |
| Lactose | 95.0mg |
| Microcrystalline Cellulose | 90.0mg |
| Cross-linked polyvinylpyrrolidone | 8.0mg |
| Magnesium Stearate | 2.0mg |
| Compression weight | 200.0mg |

The compound of the invention, microcrystalline cellulose, lactose and cross-linked polyvinylpyrrolidone are sieved through a 500 micron sieve and blended in a suitable mixer. The magnesium stearate is sieved through a 250 micron sieve and blended with the active blend. The blend is compressed into tablets using suitable punches.

| | |
|---|---|
| b) Compound of the invention | 5.0mg |
| Lactose | 165.0mg |
| Pregelatinised Starch | 20.0mg |
| Cross-linked polyvinylpyrrolidone | 8.0mg |
| Magnesium Stearate | 2.0mg |
| Compression weight | 200.0mg |

The compound of the invention, lactose and pregelatinised starch are blended together and granulated with water. The wet mass is dried and milled. The magnesium stearate and cross-linked polyvinylpyrrolidone are screened through a 250 micron sieve and blended with the granule. The resultant blend is compressed using suitable tablet punches.

Example 44

Pharmacy Example - Capsules

| | |
|---|---|
| a) Compound of the invention | 5.0mg |
| Pregelatinised Starch | 193.0mg |
| Magnesium Stearate | 2.0mg |
| Fill weight | 200.0mg |

The compound of the invention and pregelatinised starch are screened through a 500 micron mesh sieve, blended together and lubricated with magnesium stearate, (meshed through a 250 micron sieve). The blend is filled into hard gelatine capsules of a suitable size.

| | |
|---|---|
| b) Compound of the invention | 5.0mg |
| Lactose | 177.0mg |
| Polyvinylpyrrolidone | 8.0mg |
| Cross-linked polyvinylpyrrolidone | 8.0mg |
| Magnesium Stearate | 2.0mg |
| Fill weight | 200.0mg |

The compound of the invention and lactose are blended together and granulated with a solution of polyvinylpyrrolidone. The wet mass is dried and milled. The magnesium stearate and cross-linked polyvinylpyrrolidone are screened through a 250 micron sieve and blended with the granules. The resultant blend is filled into hard gelatine capsules of a suitable size.

Example 45

Pharmacy Example – Syrup

| | |
|---|---|
| a) Compound of the invention | 5.0mg |
| Hydroxypropyl Methylcellulose | 45.0mg |
| Propyl Hydroxybenzoate | 1.5mg |
| Butyl Hydroxybenzoate | 0.75mg |
| Saccharin Sodium | 5.0mg |
| Sorbitol Solution | 1.0ml |
| Suitable Buffers | qs |
| Suitable flavours | qs |
| Purified Water to | 10.ml |

The hydroxypropyl methylcellulose is dispersed in a portion of hot purified water together with the hydroxybenzoates and the solution is allowed to cool to room temperature. The saccharin sodium flavours and sorbitol solution are added to the bulk solution. The compound of the invention is dissolved in a portion of the remaining water and added to the bulk solution. Suitable buffers may be added to control the pH in the region of maximum stability. The solution is made up to volume, filtered and filled into suitable containers.

Example 46

Pharmacy Example – Injection Formulation

| | % w/v |
|---|---|
| Compound of the invention | 1.00 |
| Water for injections B.P. to | 100.00 |

Sodium chloride may be added to adjust the tonicity of the solution and the pH may be adjusted to that of maximum stability and/or to facilitate solution of the compound of the invention using dilute acid or alkali or by the addition of suitable buffer salts. Antioxidants and metal chelating salts may also be included.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

**Claims**

**1.** Compounds of formula (I)

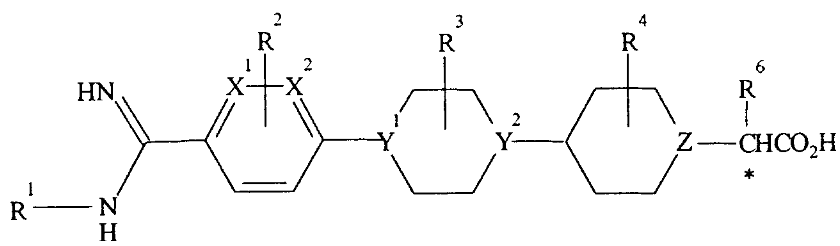

(I)

in which

$X^1$ and $Y^1$, which may be the same or different, represent CH or N;

$X^2$ represents CH or, when $X^1$ represents CH, may also represent N;

$Y^2$ represents N or, when $Y^1$ represents N, may also represent CH;
Z represents N or $N^+R^5$;
$R^1$ represents a hydrogen atom or a hydroxyl, $C_{1-4}$alkyl or 2,2,2-trifluoroethyl group;
$R^2$ represents a hydrogen atom or, when both $X^1$ and $X^2$ represent CH, may also represent a fluorine, chlorine or bromine atom or a $C_{1-4}$alkyl group;
$R^3$ represents a hydrogen atom or, when both $Y^1$ and $Y^2$ represent N, may also represent a $C_{1-4}$alkyl or hydroxymethyl group.
$R^4$ represents a hydrogen atom or, when Z represents N, $R^4$ may also represent a $C_{1-4}$alkyl group;
$R^5$ represents a $C_{1-4}$alkyl or phenyl$C_{1-4}$alkyl group;
$R^6$ represents a hydrogen atom or a $C_{1-4}$alkyl group;
and pharmaceutically acceptable derivatives thereof and salts and solvates thereof.

**2.** Compounds as claimed in Claim 1 in which both $X^1$ and $X^2$ represent CH.

**3.** Compounds as claimed in Claim 1 or 2 in which both $Y^1$ and $Y^2$ represent N.

**4.** Compounds as claimed in any one of Claims 1 to 3 in which Z represents N or $N^+C_{1-4}$alkyl.

**5.** Compounds as claimed in any one of Claims 1 to 4 in which Z represents N.

**6.** Compounds as claimed in any one of Claims 1 to 5 in which $R^1$ represents a hydrogen atom.

**7.** Compounds as claimed in any one of Claims 1 to 6 in which $R^2$, $R^3$ and $R^4$ each represent a hydrogen atom.

**8.** Compounds as claimed in any one of Claims 1 to 7 in which $R^6$ represents a hydrogen atom.

**9.** Compounds as claimed in Claim 1 in which $X^1$ and $X^2$ both represent CH; $Y^1$, $Y^2$ and Z represent N; and $R^1$, $R^2$, $R^3$, $R^4$ and $R^6$ represent a hydrogen atom.

**10.** 4-[4-[4-(Aminoiminomethyl)phenyl]-1-piperazinyl]-1-piperidineacetic acid; and physiologically acceptable salts and solvates thereof.

**11.** trans-4-[4-[4-(Aminoiminomethyl)phenyl]-1-piperazinyl]-1-[2-(1,1-dimethylethoxy)-2-oxoethyl]-1-methylpiperidinium salts, including physiologically acceptable salts and solvates thereof;
1,1-Dimethylethyl 4-[4-[4-[amino(hydroxyimino)methyl]phenyl]-1-piperazinyl]-1-piperidineacetate and physiologically acceptable salts and solvates thereof;
1,1-Dimethylethyl 4-[4-[4-[imino[(2,2,2-trifluoroethyl)amino] methyl]phenyl]-1-piperazinyl]-1-piperidineacetate and physiologically acceptable salts and solvates thereof;
1,1-Dimethylethyl 4-[4-[6-(aminoiminomethyl)-3-pyridinyl]-1-piperazinyl]-1-piperidineacetate and physiologically acceptable salts and solvates thereof;
1,1-Dimethylethyl 4-[4-[4-(aminoiminomethyl)phenyl]-2-methyl-1-piperazinyl]-1-piperidineacetate and physiologically acceptable salts and solvates thereof;
trans-4-[1-[4-(Aminoiminomethyl)phenyl)-4-piperidinyl]-1-[2-(1,1-dimethylethoxy)-2-oxoethyl]-1-methylpiperidinium salts, including physiologically acceptable salts and solvates thereof;
1,1-Dimethylethyl 4-[4-(aminoiminomethyl)phenyl][1,4'-bipiperidine]-1'-acetate and physiologically acceptable salts and solvates thereof;
1,1-Dimethylethyl 4-[4-[4-(aminoiminomethyl)phenyl]-1-piperazinyl]-1-piperidineacetate and physiologically acceptable salts and solvates thereof;
1,1-Dimethylethyl 4-[4-[4-(aminoiminomethyl)-2-bromophenyl]-1-piperazinyl]-1-piperidineacetate and physiologically acceptable salts and solvates thereof;
1,1-Dimethylethyl 4-[4-[4-(aminoiminomethyl)-2-methylphenyl]-1-piperazinyl]-1-piperidineacetate and physiologically acceptable salts and solvates thereof;
Ethyl 4-[4-[4-(aminoiminomethyl)phenyl]-1-piperazinyl]-α-methyl-1-piperidineacetate and physiologically acceptable salts and solvates thereof;
1,1-Dimethylethyl 1'-[4-(aminoiminomethyl)phenyl][4,4'-piperidine]-1-acetate and physiologically acceptable salts and solvates thereof;
trans-4-[4-[4-(Aminoiminomethyl)phenyl]-1-piperazinyl]-1-[2-(1,1-dimethylethoxy)-2-oxoethyl] – 1 – (phenylmethyl)piperidinium salts, including physiologically acceptable salts and solvates thereof;
cis – 4 – [4 – [4 – (Aminoiminomethyl)phenyl] – 1 – piperazinyl] – 1 – [2 – (1,1 – dimethylethoxy) – 2 – oxoethyl] – 1 – (phenylmethyl)piperidinium salts, including physiologically acceptable salts and solvates thereof;
cis – 1,1 – Dimethylethyl 4 – [4 – [4 – [amino(hydroxyimino)methyl]phenyl] – 1 – piperazinyl] – 3 – methyl – 1 – piperidineacetate and physiologically acceptable salts and solvates thereof;
trans – 4 – [4 – [4 – (Aminoiminomethyl)phenyl] – 1 – piperazinyl] – 1 – (2 – hydroxy – 2 – oxoethyl) – 1 – methylpiperidinium salts, including physiologically acceptable salts and solvates thereof;
4 – [4 – [4 – (Amino(hydroxyimino)methyl]phenyl] – 1 – piperazinyl] – 1 – piperidineacetic acid and physiologically acceptable salts and solvates thereof;
4 – [4 – [4 – (Imino[(2,2,2 – trifluoroethyl)amino]methyl]phenyl] – 1 – piperazinyl] – 1 – piperidineacetic acid and physiologically acceptable salts and solvates thereof;
4 – [4 – [6 – (Aminoiminomethyl) – 3 – pyridinyl] – 1 – piperazinyl] – 1 – piperidineacetic acid and physiologically acceptable salts and solvates thereof;
4 – [4 – [4 – (Aminoiminomethyl)phenyl] – 2 – methyl – 1 – piperazinyl] – 1 – piperidineacetic acid and physiologically acceptable salts and solvates thereof;
trans – 4 – [1 – [4 – (Aminoiminomethyl)phenyl] – 4 – piperidinyl] – 1 – (2 – hydroxy – 2 – oxoethyl) – 1 – methylpiperidinium salts, including physiologically acceptable salts and solvates thereof;
4 – [4 – (Aminoiminomethyl)phenyl][1,4' – bipiperidine] – 1' – acetic acid and physiologically acceptable salts and solvates thereof;
Ethyl 4 – [4 – [4 – (aminoiminomethyl)phenyl] – 1 – piperazinyl] – 1 – piperidineacetate and physiologically acceptable salts and solvates thereof;
4 – [4 – [4 – (Aminoiminomethyl) – 2 – bromophenyl] – 1 – piperazinyl] – 1 – piperidineacetic acid and physiologically acceptable salts and solvates thereof;
4 – [4 – [4 – (Aminoiminomethyl) – 2 – methylphenyl] – 1 – piperazinyl] – 1 – piperidineacetic acid and physiologically acceptable salts and solvates thereof;
4 – [4 – [4 – (Aminoiminomethyl)phenyl] – 1 – piperazinyl] – α – methyl – 1 – piperidineacetic acid, in the form of a racemic mixture or a single enantiomer, and physiologically acceptable salts and solvates thereof;
1' – [4 – (Aminoiminomethyl)phenyl][4,4' – bipiperidine] – 1 – acetic acid and physiologically acceptable salts and solvates thereof;
trans – 4 – [4 – [4 – (Aminoiminomethyl)phenyl] – 1 – piperazinyl] – 1 – (2 – hydroxy – 2 – oxoethyl) – 1 – (phenylmethyl)piperidinium salts, including physiologically acceptable salts and solvates thereof;
cis – 4 – [4 – [4 – (Aminoiminomethyl)phenyl] – 1 – piperazinyl] – 1 – (2 – hydroxy – 2 – oxoethyl) – 1 – (phenylmethyl)piperidinum salts, including physiologically acceptable salts and solvates thereof;
cis – 4 – [4 – [4 – (Aminoiminomethyl)phenyl] – 1 – piperazinyl] – 3 – methyl – 1 – piperidineacetic acid and physiologically acceptable salts and solvates thereof;
1,1 – Dimethylethyl 4 – [4 – [4 – [[(ethoxycarbonyl)amino]iminomethyl]phenyl] – 1 – piperazinyl] – 1 – piperidineacetate and physiologically acceptable salts and solvates thereof;
4 – [4 – [4 – [[(Ethoxycarbonyl)amino]iminomethyl]phenyl] – 1 – piperazinyl] – piperidineacetic acid and physiologically acceptable salts and solvates thereof;
1,1 – Dimethylethyl 4 – [4 – [4 – [imino[(methoxycarbonyl)amino] methyl]phenyl] – 1 – piperazinyl] – 1 – piperidineacetate and physiologically acceptable salts and solvates thereof;
4 – [4 – [4 – [Imino[(methoxycarbonyl)amino]methyl]phenyl] – 1 – piperazinyl] – 1 – piperidineacetic acid and physiologically acceptable salts and solvates thereof;
1,1 – Dimethylethyl 4 – [4 – [4[[(diethoxyphosphinyl)amino] iminomethyl]phenyl] – 1 – piperazinyl] – 1 – piperidineacetate and physiologically acceptable salts and solvates thereof;
4 – [4 – [4 – [[(Diethylphosphinyl)amino]iminomethyl]phenyl] – 1 – piperazinyl] – 1 – piperidineacetic acid and physiologically acceptable salts and solvates thereof;
1,1 – Dimethylethyl 4 – [4 – [4 – [(benzoylamino)iminomethyl]phenyl] – 1 – piperazinyl] – 1 – piperidineacetate and physiologically acceptable salts and solvates thereof;
4 – [4 – [4 – [(Benzoylamino)iminomethyl]phenyl] – 1 – piperazinyl] – 1 – piperidineacetic acid and physiologically acceptable salts and solvates thereof;
1,1 – Dimethylethyl 4 – [4 – [5 – [amino(hydroxyimino)methyl] – 2 – pyridinyl] – 1 – piperazinyl] – 1 – piperidineacetate and physiologically acceptable salts and solvates thereof;
1,1 – Dimethylethyl 4 – [4 – [5 – (aminoiminomethyl) – 2 – pyridinyl] – 1 – piperazinyl] – 1 – piperidineacetate and physiologically acceptable salts and solvates thereof; and

4-[4-[5-(Aminoiminomethyl)-2-pyridinyl]-1-piperazinyl]-1-piperidineacetic acid and physiologically acceptable salts and solvates thereof.

**12.** Compounds as claimed in any of Claims 1 to 11 wherein the compound of formula (I) is in the form of a hydrochloride, hydrobromide, sulphate, phosphate, benzoate, naphthoate, hydroxynaphthoate, p-toluenesulphonate, methanesulphonate, sulphamate, ascorbate, tartrate, salicylate, succinate, lactate, glutarate, glutaconate, acetate, tricarballylate, citrate, fumarate, maleate or sodium salt.

**13.** A process for the preparation of compounds of formula (I) as defined in any of Claims 1 to 12 or a physiologically acceptable salt or solvate thereof; which comprises:

(A) for the preparation of compounds of formula (I) in which $R^1$ represents a hydrogen atom; alkylating a compound of formula (II)

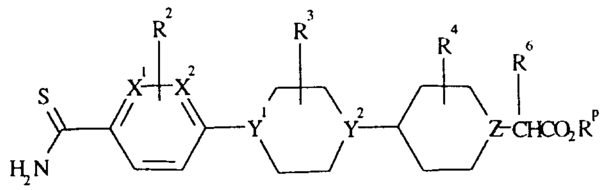

(II)

(wherein $R^p$ represents a protecting group) followed by reaction with a source of ammonia; or

(B) for the preparation of a compound of formula (I) in which $R^1$ represents a hydroxyl group; treating a compound of formula (III)

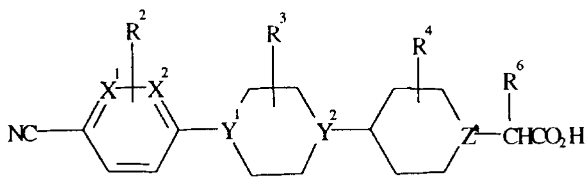

(III)

or a protected derivative thereof with hydroxylamine or an acid addition salt thereof; or

(C) for the preparation of a compound of formula (I) in which $R^1$ represents a hydroxyl, $C_{1-4}$alkyl or 2,2,2-trifluoroethyl group, forming a thioimidate from a compound of formula (II) followed by reaction with an amine $R^1NH_2$ (where $R^1$ represents hydroxy, $C_{1-4}$alkyl or 2,2,2-trilluoroethyl); or

(D) forming an imidate from a compound of formula (IV)

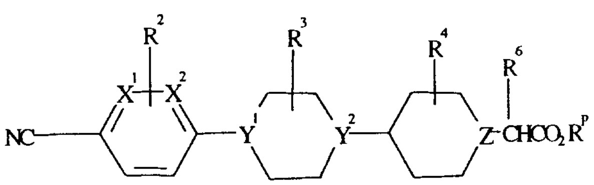

(IV)

(wherein $R^p$ represents a protecting group) followed by treatment with a source of ammonia or an amine $R^1NH_2$ (where $R^1$ is hydroxy, $C_{1-4}$alkyl or 2,2,2-trifluoroethyl); or

(E) interconversion of a compound of formula (I) into another compound of formula (I); or

(F) for the preparation of a compound of formula (I) in which $R^1$ represents a hydrogen, $C_{1-4}$alkyl or 2,2,2-trifuloroethyl and Z represents N;

hydrogenating a compound of formula (XII)

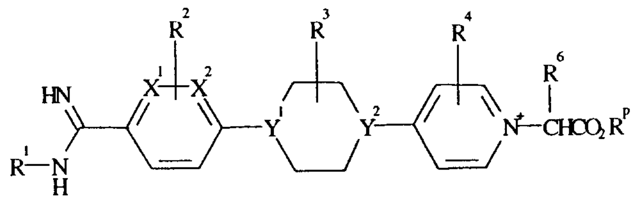 (XII)

(where $R^1$ is hydrogen, $C_{1-4}$alkyl or 2,2,2 – trifluoroethyl); or

(G) optionally removing any protecting groups from a protected derivative of formula (I); with the formation of a pharmaceutically acceptable derivative, salt formation and resolution as optional steps subsequent to any of processes (A) to (G).

**14.** A pharmaceutical composition comprising a compound of formula (I) as defined in any one of Claims 1 to 12 or a physiologically acceptable salt or solvate thereof together with at least one physiologically acceptable carrier or excipient.

**15.** A compound of formula (I) as defined in any of Claims 1 to 12 or a physiologically acceptable salt or solvate thereof for use in human or veterinary medicine.

**16.** The use of a compound of formula (I) or a physiologically acceptable salt or solvate thereof as defined in any of Claims 1 to 12 for the manufacture of a medicament for the treatment or phrophylaxis of thrombotic disorders.

**17.** A method of treating a human or animal subject suffering from or susceptible to a thrombotic disorder, which method comprises administering to said subject an effective amount of a compound of formula (I) as defined in any of claims 1 to 12 or a physiologically acceptable salt or solvate thereof.